# EUROPEAN PATENT SPECIFICATION

(11) **EP 2 483 255 B1**
(45) Date of publication and mention of the grant of the patent: **16.10.2013**
(21) Application number: 11806295.9
(22) Date of filing: 13.07.2011
(51) Int. Cl.: C07D 265/30, C07D 413/12, C07D 417/12, C07D 471/04, C07D 487/04, A61K 31/5377, A61K 31/553, A61P 25/00, A61P 9/00, C07D 413/14

(54) **OXAZINE DERIVATIVES AND THEIR USE IN THE TREATMENT OF NEUROLOGICAL DISORDERS**
OXAZINDERIVATE UND IHRE VERWENDUNG BEI DER BEHANDLUNG VON NERVENERKRANKUNGEN
DÉRIVÉS DE L'OXAZINE ET LEUR UTILISATION DANS LE TRAITEMENT DE TROUBLES NEUROLOGIQUES

(30) Priority: 21.01.2011 US 201161435088 P; 12.01.2011 US 201161432058 P; 23.07.2010 WO PCT/EP2010/060718; 13.07.2010 US 201061363702 P
(43) Date of publication of application: 08.08.2012
(73) Proprietor: Novartis AG, 4056 Basel (CH)
(72) Inventor: BADIGER, Sangamesh, Bangalore 560100 (IN); CHEBROLU, Murali, Bangalore 560100 (IN); FREDERIKSEN, Mathias, CH-4002 Basel (CH); HOLZER, Philipp, CH-4002 Basel (CH); HURTH, Konstanze, CH-4002 Basel (CH); LI, Lei, Changshu Jiangsu 215537 (CN); LIU, Hui, Changshu Jiangsu 215537 (CN); LUEOEND, Rainer Martin, CH-4002 Basel (CH); MACHAUER, Rainer, CH-4002 Basel (CH); MOEBITZ, Henrik, CH-4002 Basel (CH); NEUMANN, Ulf, CH-4002 Basel (CH); RAMOS, Rita, CH-4002 Basel (CH); RUEEGER, Heinrich, CH-4002 Basel (CH); SCHAEFER, Michael, CH-4002 Basel (CH); TINTELNOT-BLOMLEY, Marina, CH-4002 Basel (CH); VEENSTRA, Siem Jacob, CH-4002 Basel (CH); VOEGTLE, Markus, CH-4002 Basel (CH); XIONG, Xin, Changshu Jiangsu 215537 (CN)
(74) Representative: Campbell, Lachlan Clive
(86) International application number: PCT/CN2011/077119
(87) International publication number: WO 2012/006953

(56) References cited:
- EP-A1- 1 942 105
- WO-A1-2007/049532
- WO-A1-2008/133273
- WO-A1-2011/009943
- WO-A1-2011/020806
- WO-A2-2006/034093
- US-A- 5 691 336

## Description

Alzheimer's Disease is a devastating neurodegenerative disorder. Its sporadic forms affect an elderly population (sharp increase in incidence at >75 years of age), in addition, there are various familial forms with an onset of the disease in the fourth or fifth decade of life. Pathologically, it is characterized by the presence of extracellular senile plaques, and intracellular neurofibrillar tangles in patient's brains. The core constituent of the senile plaques are small, 4 kDa amyloid peptides. They are generated by the proteolytic processing of a large transmembrane protein, amyloid precursor protein (APP). Cleavage of APP by beta-secretase (BACE-1) releases the soluble APP-beta fragment, while the 99-amino acid long C-terminus remains tethered to the membrane. This C-terminal fragment is subsequently proteolytically processed by gamma-secretase (an membrane multi-enzyme complex) to generate amyloid peptides of various length, predominantly 40 and 42 amino acids long (Hardy J, Selkoe DJ (2002) Science; 297 (5580):353-356).

If, under pathologic conditions, the generation of these peptides occurs at an increased rate, or if their removal from the brain is disturbed, increased brain amyloid peptide concentrations leads to the formation of oligomers, fibrils and eventually plaques (Farris W, et al (2007) Am.J. Pathol.; 171 (1):241-251). It has been shown, that deposition of amyloid peptides and plaques in the brain is the first measurable event in the pathogenesis of Alzheimers Disease, and that it is the trigger for loss of synapses, synaptic contacts, and neurons (Grimmer T, et al (2009) Neurobiology of Aging; 30 (12):1902-1909). Brain atrophy caused by massive neuron loss is followed by impairments in cognition, memory, orientation and the ability to perform the tasks of daily living, i.e. clinically manifest dementia (Okello A, et al (2009) Neurology; 73 (10):754-760).

BACE-1, also known as Asp2 or Memapsin 2, is a transmembrane aspartic protease highly expressed in neurons. It co-localizes with its substrate APP in Golgi and endocytic compartments (Willem M, Lammich S, Haass C (2009) Semin.Cell Dev.Biol; 20 (2):175-182). Knock-out studies in mice have demonstrated the absence of amyloid peptide formation, while the animals are healthy and fertile (Ohno M, et al (2007) Neurobiol.Dis.; 26 (1):134-145). Genetic ablation of BACE-1 in APP-overexpressing mice has demonstrated absence of plaque formation, and the reverse of cognitive deficits (Ohno M, et al (2004) Neuron; 41 (1):27-33). BACE-1 levels are elevated in the brains of sporadic Alzheimer's Disease patients (Hampel H, Shen Y (2009) Scand. J. Clin. Lab. Invest.; 69 (1):8-12).

Taken together, these findings suggest that the inhibition of BACE-1 may be a favourable therapeutic strategy for Alzheimer's Disease.

WO 2008/133273 and WO 2007/049532 disclose BACE inhibitors.

The present invention relates to novel oxazine derivatives having BACE inhibitory activity, to their preparation, to their medical use and to medicaments comprising them.

The invention relates to a compound of the formula 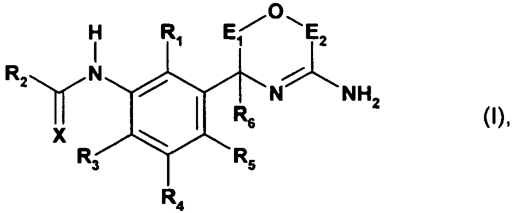 in which X, E₁, E₂, R₁, R₂, R₃, R₄, R₅, and R₆, are so defined as to provide a compound selected from:
5-cyano-pyridine-2-carboxylic acid [3-(5-amino-3-difluoromethyl-3,6-dihydro-2H-[1,4]oxazin-3-yl)-4-fluoro-phenyl]-amide;
5-cyano-3-methyl-pyridine-2-carboxylic acid [3-(5-amino-3-difluoromethyl-3,6-dihydro-2H-[1,4]oxazin-3-yl)-4-fluoro-phenyl]-amide;
5-cyano-3-methyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide; and
pharmaceutically acceptable salts thereof.

In a first aspect, there is therefore provided a compound selected from the group consisting of: 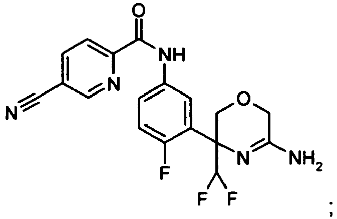 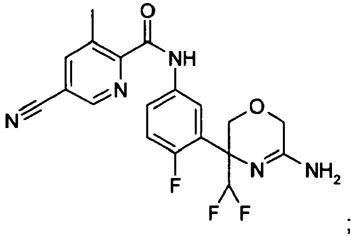 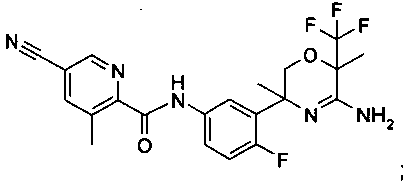 and pharmaceutically acceptable salts thereof.

In one embodiment, there is provided a compound, or a pharmaceutically acceptable salt thereof, having the following formula

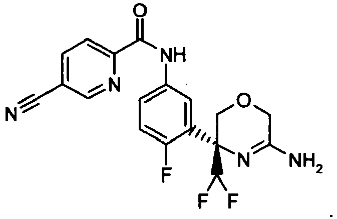

In another embodiment, there is provided a compound, or a pharmaceutically acceptable salt thereof, having the following formula

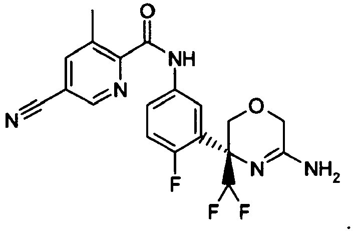

In another embodiment, there is provided a compound, or a pharmaceutically acceptable salt thereof, having the following formula

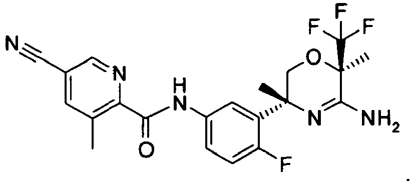

In another aspect, there is provided a pharmaceutical composition comprising a compound as defined above, or a pharmaceutically acceptable salt thereof, as active ingredient and a pharmaceutical carrier or diluent.

On account of one or more than one asymmetrical carbon atom, which may be present in a compound of the formula I, a corresponding compound of the formula I may exist in pure optically active form or in the form of a mixture of optical isomers, e. g. in the form of a racemic mixture. All of such pure optical isomers and all of their mixtures, including the racemic mixtures, are part of the present invention.

As used herein, the term "isomers" refers to different compounds that have the same molecular formula but differ in arrangement and configuration of the atoms. Also as used herein, the term "an optical isomer" or "a stereoisomer" refers to any of the various stereo isomeric configurations which may exist for a given compound of the present invention and includes geometric isomers. It is understood that a substituent may be attached at a chiral center of a carbon atom. Therefore, the invention includes enantiomers, diastereomers or racemates of the compound. "Enantiomers" are a pair of stereoisomers that are non-superimposable mirror images of each other. A 1:1 mixture of a pair of enantiomers is a "racemic" mixture. The term is used to designate a racemic mixture where appropriate. "Diastereoisomers" are stereoisomers that have at least two asymmetric atoms, but which are not mirror-images of each other. The absolute stereochemistry is specified according to the Cahn- Ingold- Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers or axes and may thus give rise to enantiomers, diastereomers, and other stereoisomeric forms that may be defined, in terms of absolute stereochemistry, as (R)- or (S)-. The present invention is meant to include all such possible isomers, including racemic mixtures, optically pure forms and intermediate mixtures. Optically active (R)- and (S)- isomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration.

A compound of the formula I may exist in tautomeric form. All such tautomers are part of the present invention.

In yet a further embodiment, the invention relates to 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide in free form. In yet a further embodiment, the invention relates to 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide in pharmaceutically acceptable salt form. In yet a further embodiment, the invention relates to 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide in hydrochloride salt form.

In yet a further embodiment, the invention relates to 5-cyano-pyridine-2-carboxylic acid [3-((R)-5-amino-3-difluoromethyl-3,6-dihydro-2H-[1,4]oxazin-3-yl)-4-fluoro-phenyl]-amide in free form. In yet a further embodiment, the invention relates to 5-cyano-pyridine-2-carboxylic acid [3-((R)-5-amino-3-difluoromethyl-3,6-dihydro-2H-[1,4]oxazin-3-yl)-4-fluoro-phenyl]-amide in pharmaceutically acceptable salt form. In yet a further embodiment, the invention relates to 5-cyano-pyridine-2-carboxylic acid [3-((R)-5-amino-3-difluoromethyl-3,6-dihydro-2H-[1,4]oxazin-3-yl)-4-fluoro-phenyl]-amide in hydrochloride salt form.

In yet a further embodiment, the invention relates to 5-cyano-3-methyl-pyridine-2-carboxylic acid [3-((R)-5-amino-3-difluoromethyl-3,6-dihydro-2H-[1,4]oxazin-3-yl)-4-fluoro-phenyl]-amide in free form. In yet a further embodiment, the invention relates to 5-cyano-3-methylpyridine-2-carboxylic acid [3-((R)-5-amino-3-difluoromethyl-3,6-dihydro-2H-[1,4]oxazin-3-yl)-4-fluoro-phenyl]-amide in pharmaceutically acceptable salt form. In yet a further embodiment, the invention relates to 5-cyano-3-methyl-pyridine-2-carboxylic acid [3-((R)-5-amino-3-difluoromethyl-3,6-dihydro-2H-[1,4]oxazin-3-yl)-4-fluoro-phenyl]-amide in hydrochloride salt form. In yet a further embodiment, the invention relates 5-cyano-3-methylpyridine-2-carboxylic acid [3-((R)-5-amino-3-difluoromethyl-3,6-dihydro-2H-[1,4]oxazin-3-yl)-4-fluoro-phenyl]-amide hydrochloride hydrate.

As used herein, the terms "salt" or "salts" refers to an acid addition or base addition salt of a compound of the invention. "Salts" include in particular "pharmaceutical acceptable salts". The term "pharmaceutically acceptable salts" refers to salts that retain the biological effectiveness and properties of the compounds of this invention and, which typically are not biologically or otherwise undesirable. In many cases, the compounds of the present invention are capable of forming acid and/or base salts by virtue of the presence of amino and/or carboxyl groups or groups similar thereto.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfomate, chloride/hydrochloride, chlortheophyllonate, citrate, ethandisulfonate, fumarate, gluceptate, gluconate, glucuronate, hippurate, , hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts. Inorganic acids from which salts can be derived include, for example, hydrochloric acid, hydrobromic acid, sulfuric acid, nitric acid and phosphoric acid. Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid and sulfosalicylic acid. Pharmaceutically acceptable base addition salts can be formed with inorganic and organic bases. Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines and basic ion exchange resins. Certain organic amines include isopropylamine, benzathine, cholinate, diethanolamine, diethylamine, lysine, meglumine, piperazine and tromethamine.

The pharmaceutically acceptable salts of the present invention can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

When both a basic group and an acid group are present in the same molecule, the compounds of the present invention may also form internal salts, e.g., zwitterionic molecules.

Pro-drugs of the compounds of the present invention convert *in vivo* to the compounds of the present invention. A pro-drug is an active or inactive compound that is modified chemically through *in vivo* physiological action, such as hydrolysis, metabolism and the like, into a compound of this invention following administration of the prodrug to a subject. The suitability and techniques involved in making and using pro-drugs are well known by those skilled in the art. Prodrugs can be conceptually divided into two non-exclusive categories, bioprecursor prodrugs and carrier prodrugs. See The Practice of Medicinal Chemistry, Ch. 31-32 (Ed. Wermuth, Academic Press, San Diego, Calif., 2001). Generally, bioprecursor prodrugs are compounds, which are inactive or have low activity compared to the corresponding active drug compound, that contain one or more protective groups and are converted to an active form by metabolism or solvolysis. Both the active drug form and any released metabolic products should have acceptably low toxicity.

Carrier prodrugs are drug compounds that contain a transport moiety, *e.g*., that improve uptake and/or localized delivery to a site(s) of action. Desirably for such a carrier prodrug, the linkage between the drug moiety and the transport moiety is a covalent bond, the prodrug is inactive or less active than the drug compound, and any released transport moiety is acceptably non-toxic. For prodrugs where the transport moiety is intended to enhance uptake, typically the release of the transport moiety should be rapid. In other cases, it is desirable to utilize a moiety that provides slow release, e.g., certain polymers or other moieties, such as cyclodextrins. Carrier prodrugs can, for example, be used to improve one or more of the following properties: increased lipophilicity, increased duration of pharmacological effects, increased site-specificity, decreased toxicity and adverse reactions, and/or improvement in drug formulation (e.g., stability, water solubility, suppression of an undesirable organoleptic or physiochemical property). For example, lipophilicity can be increased by esterification of (a) hydroxyl groups with lipophilic carboxylic acids (e.g., a carboxylic acid having at least one lipophilic moiety), or (b) carboxylic acid groups with lipophilic alcohols (e.g., an alcohol having at least one lipophilic moiety, for example aliphatic alcohols).

Exemplary prodrugs are, *e.g*., esters of free carboxylic acids and *S*-acyl derivatives of thiols and O-acyl derivatives of alcohols or phenols, wherein acyl has a meaning as defined herein. Suitable prodrugs are often pharmaceutically acceptable ester derivatives convertible by solvolysis under physiological conditions to the parent carboxylic acid, *e.g*., lower alkyl esters, cycloalkyl esters, lower alkenyl esters, benzyl esters, mono- or di-substituted lower alkyl esters, such as the ω-(amino, mono- or di-lower alkylamino, carboxy, lower alkoxycarbonyl)-lower alkyl esters, the α-(lower alkanoyloxy, lower alkoxycarbonyl or di-lower alkylaminocarbonyl)-lower alkyl esters, such as the pivaloyloxymethyl ester and the like conventionally used in the art. In addition, amines have been masked as arylcarbonyloxymethyl substituted derivatives which are cleaved by esterases *in vivo* releasing the free drug and formaldehyde (Bundgaard, J. Med. Chem. 2503 (1989)). Moreover, drugs containing an acidic NH group, such as imidazole, imide, indole and the like, have been masked with N-acyloxymethyl groups (Bundgaard, Design of Prodrugs, Elsevier (1985)). Hydroxy groups have been masked as esters and ethers. EP 039,051 (Sloan and Little) discloses Mannich-base hydroxamic acid prodrugs, their preparation and use.

Furthermore, the compounds of the present invention, including their salts, can also be obtained in the form of their hydrates, or include other solvents used for their crystallization. The compounds of the present invention may inherently or by design form solvates with pharmaceutically acceptable solvents (including water); therefore, it is intended that the invention embrace both solvated and unsolvated forms. The term "solvate" refers to a molecular complex of a compound of the present invention (including pharmaceutically acceptable salts thereof) with one or more solvent molecules. Such solvent molecules are those commonly used in the pharmaceutical art, which are known to be innocuous to the recipient, e.g., water, ethanol, and the like. The term "hydrate" refers to the complex where the solvent molecule is water.

The compounds of the present invention, including salts, hydrates and solvates thereof, may inherently or by design form polymorphs.

The present invention includes all pharmaceutically acceptable isotope-labeled compounds of the formula I, wherein one or more than one atom is / are replaced by one or more than one atom having the same atomic number as, but an atomic mass different from, the one(s) usually found in nature. Examples of such isotopes are those of carbon, such as ¹¹C, ¹³C or ¹⁴C, chlorine, such as ³⁶Cl, fluorine, such as ¹⁸F, bromine, such as ⁷⁶Br, hydrogen, such as ²H or ³H, iodine, such as ¹²³I,¹²⁴I, ¹²⁵I or ¹³¹I, nitrogen, such as ¹³N or ¹⁵N, oxygen, such as ¹⁵O, ¹⁷O or ¹⁸O, phosphorus, such as ³²P, or sulphur, such as ³⁵S. An isotope-labeled compound of the formula I can be prepared by a process analogous to those described in the Examples or by a conventional technique known to those skilled in the art using an appropriate isotopically-labeled reagent or starting material. The incorporation of a heavier isotope, such as ²H (D), may provide greater metabolic stability to a compound of the formula I, which may result in, for example, an increased *in vivo*-half-life of the compound or in reduced dosage requirements. Certain isotope-labeled compounds of the formula I, for example those incorporating a radioactive isotope, such as ³H or ¹⁴C, may be used in drug or substrate-tissue distribution studies. Compounds of the formula I with a positron emitting isotope, such as ¹¹C, ¹⁸F, ¹³N or ¹⁵O, may be useful in positron emission tomography (PET) or single photon emission computed tomography (SPECT) studies, e. g. to examine substrate-receptor occupancies.

Pharmaceutically acceptable solvates in accordance with the invention include those wherein the solvent of crystallization may be isotopically substituted, e.g. D2O, d6-acetone, d6-DMSO.

Compounds of the invention, i.e. compounds of formula (I) that contain groups capable of acting as donors and/or acceptors.for hydrogen bonds may be capable of forming co-crystals with suitable co-crystal formers. These co-crystals may be prepared from compounds of formula (I) by known co-crystal forming procedures. Such procedures include grinding, heating, co-subliming, co-melting, or contacting in solution compounds of formula (I) with the co-crystal former under crystallization conditions and isolating co-crystals thereby formed. Suitable co-crystal formers include those described in WO 2004/078163. Hence the invention further provides co-crystals comprising a compound of formula (I).

In another embodiment, the invention relates to a compound of the invention, or a pharmaceutically acceptable salt thereof, which is selected from:
5-cyano-3-methyl-pyridine-2-carboxylic acid [3-(5-amino-3,6-dimethyl-6-trifluoro-methyl-3,6-dihydro-2H-[1,4]oxazin-3-yl)-4-fluoro-phenyl]-amide;
5-cyano-pyridine-2-carboxylic acid [3-(5-amino-3-difluoromethyl-3,6-dihydro-2H-[1,4]oxazin-3-yl)-4-fluoro-phenyl]-amide; and
5-cyano-3-methyl-pyridine-2-carboxylic acid [3-(5-amino-3-difluoromethyl-3,6-dihydro-2H-[1,4]oxazin-3-yl)-4-fluoro-phenyl]-amide.

In another embodiment, the invention relates to a compound of the invention, or a pharmaceutically acceptable salt thereof, which is selected from:
5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-cyano-pyridine-2-carboxylic acid [3-((R)-5-amino-3-difluoromethyl-3,6-dihydro-2H-[1,4]oxazin-3-yl)-4-fluoro-phenyl]-amide; and
5-cyano-3-methyl-pyridine-2-carboxylic acid [3-((R)-5-amino-3-difluoromethyl-3,6-dihydro-2H-[1,4]oxazin-3-yl)-4-fluoro-phenyl]-amide.

In one embodiment, the invention relates to 5-cyano-pyridine-2-carboxylic acid [3-(5-amino-3-difluoromethyl-3,6-dihydro-2H-[1,4]oxazin-3-yl)-4-fluoro-phenyl]-amide, or a pharmaceutically acceptable salt thereof. In another embodiment, the invention relates to 5-cyano-pyridine-2-carboxylic acid [3-((R)-5-amino-3-difluoromethyl-3,6-dihydro-2H-[1,4]oxazin-3-yl)-4-fluoro-phenyl]-amide, or a pharmaceutically acceptable salt thereof. In yet another embodiment, the invention relates to 5-cyano-pyridine-2-carboxylic acid [3-((S)-5-amino-3-difluoromethyl-3,6-dihydro-2H-[1,4]oxazin-3-yl)-4-fluoro-phenyl]-amide, or a pharmaceutically acceptable salt thereof.

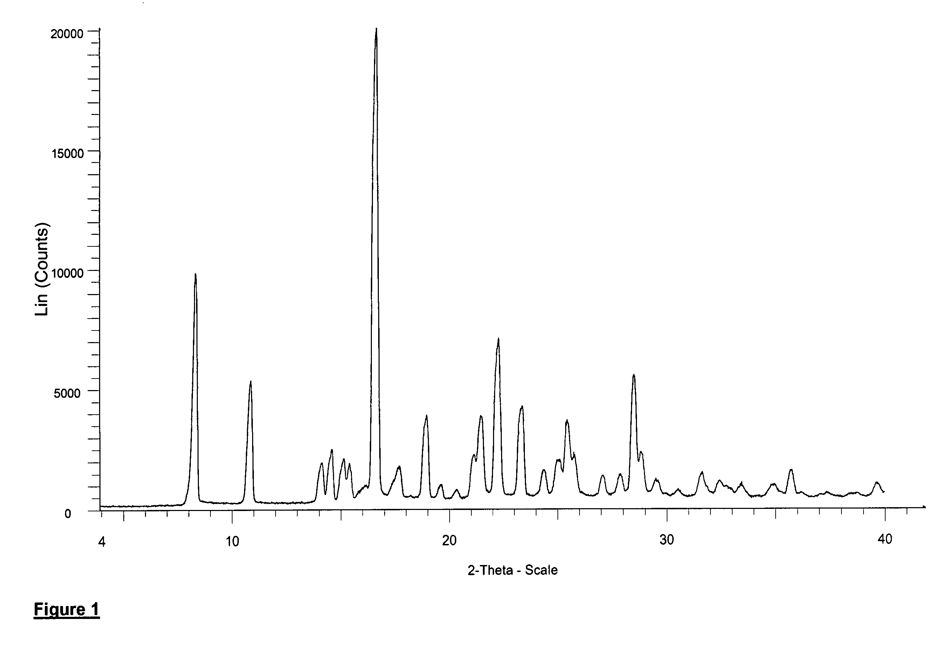

In a more focused aspect, the invention relates to a crystalline form of 5-cyano-pyridine-2-carboxylic acid [3-((R)-5-amino-3-difluoromethyl-3,6-dihydro-2H-[1,4]oxazin-3-yl)-4-fluorophenyl]-amide, or a pharmaceutically acceptable salt thereof. In another embodiment, the invention relates to a crystalline form of of 5-cyano-pyridine-2-carboxylic acid [3-((R)-5-amino-3-difluoromethyl-3,6-dihydro-2H-[1,4]oxazin-3-yl)-4-fluoro-phenyl]-amide which has an X-ray powder diffraction pattern with at least one, two or three peaks having angle of refraction 2 theta (θ) values selected from 8.3, 10.8, 16.6, 18.9, 21.5, 22.2, 23.3, 25.4 and 28.5 when measured using CuK_{α} radiation, more particularly wherein said values may be plus or minus 0.2° 2θ. In a further embodiment, the invention relates to a crystalline form of 5-cyano-pyridine-2-carboxylic acid [3-((R)-5-amino-3-difluoromethyl-3,6-dihydro-2H-[1,4]oxazin-3-yl)-4-fluoro-phenyl]-amide which has an X-ray powder diffraction pattern substantially the same as the X-ray powder diffraction pattern shown in Figure 1 when measured using CuK_{α} radiation. For details see Example 152.

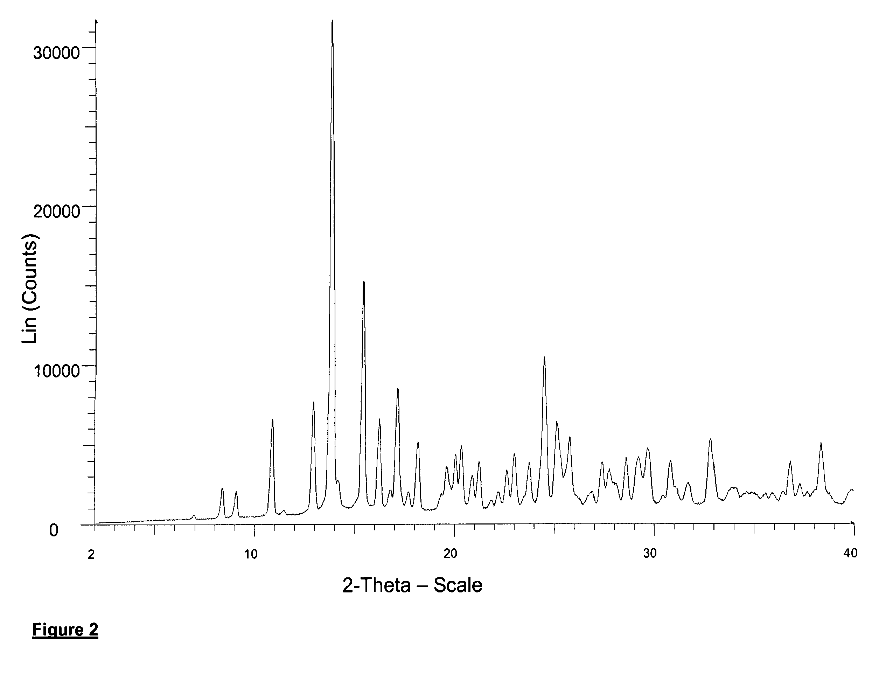

In a more focused aspect, the invention relates to a crystalline form of 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide. In another embodiment, the invention relates to a crystalline form of 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide which has an X-ray powder diffraction pattern with at least one, two or three peaks having angle of refraction 2 theta (θ) values selected from 8.3, 9.0, 10.9, 12.9, 13.9, 15.4, 16.2, 17.1, 18.2, and 24.5° when measured using CuK_{α} radiation, wherein said values are plus or minus 0.2° 2θ. In a further embodiment, the invention relates to a crystalline form of 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide which has an X-ray powder diffraction pattern substantially the same as the X-ray powder diffraction pattern shown in Figure 2 when measured using CuK_{α} radiation. For details see Example 72b. In yet a further embodiment, the invention relates to a crystalline form of 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluorophenyl]-amide in association with at least one pharmaceutically acceptable carrier or diluent.

In a more focused aspect, the invention relates to a crystalline form of 5-cyano-3-methyl-pyridine-2-carboxylic acid [3-((R)-5-amino-3-difluoromethyl-3,6-dihydro-2H-[1,4]oxazin-3-yl)-4-fluoro-phenyl]-amide. In one embodiment, the invention relates to a crystalline form of 5-cyano-3-methyl-pyridine-2-carboxylic acid [3-((R)-5-amino-3-difluoromethyl-3,6-dihydro-2H-[1,4]oxazin-3-yl)-4-fluoro-phenyl]-amide in association with at least one pharmaceutically acceptable carrier or diluent.

A process for the preparation of a compound of the formula I, in free form or in salt form, comprises
a) the reaction of a compound of the formula 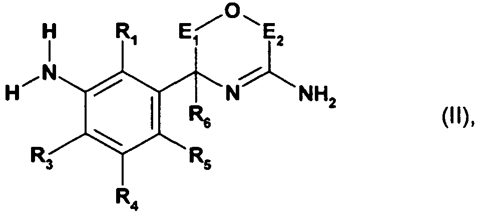 in which R₁, R₃, R₄, R₅, R₆, E₁ and E₂ are as defined for the formula I, in free form or in salt form, with a compound of the formula 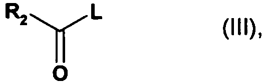 in which R₂ is as defined for the formula I and L is a leaving group, in free form or in salt form,
b) the reaction of a compound of the formula 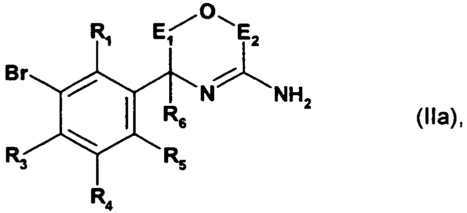 in which R₁, R₃, R₄, R₅, R₆, E₁ and E₂ are as defined for the formula I, in free form or in salt form, with a compound of the formula 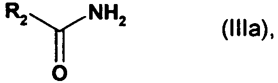 in which R₂ is as defined for the formula I, in free form or in salt form,
c) the optional reduction, oxidation or other functionalisation of the resulting compound,
d) the cleavage of any protecting group(s) optionally present and
e) the recovery of the so obtainable compound of the formula I in free form or in salt form.

The reactions can be effected according to conventional methods, for example as described in the Examples.

The working-up of the reaction mixtures and the purification of the compounds thus obtainable may be carried out in accordance with known procedures.

Salts may be prepared from free compounds in known manner, and vice-versa.

Compounds of the formula I can also be prepared by further conventional processes, e. g. as described in the Examples.

The starting materials of the formulae II and III are known, may be prepared according to conventional procedures starting from known compounds, may be prepared from known compounds as described in the Examples or may be prepared using procedures analogous to those described in the Examples.

Compounds of the formula I, in free form or in pharmaceutically acceptable salt form, hereinafter often referred to as "agents of the invention", exhibit valuable pharmacological properties, when tested *in vitro* or *in vivo,* and are, therefore, useful in medicaments.

E. g., agents of the invention are inhibitors of aspartic proteases and can be used for the treatment or prevention of a condition, disease or disorder involving processing by such enzymes. Particularly, agents of the invention inhibit beta-secretase and, thus, the generation of beta-amyloid and the subsequent aggregation into oligomers and fibrils.

The inhibiting properties of an agent of the invention towards proteases can be evaluated in tests as described hereinafter.

### Test 1: Inhibition of human BACE-1

Recombinant BACE-1 (extracellular domain, expressed in baculovirus and purified using standard methods) at 0.1 to 10 nM concentrations is incubated with the test compound at various concentrations for 1 hour at room temperature in 10 to 100 mM acetate buffer, pH 4.5, containing 0.1 % CHAPS. Synthetic fluorescence-quenched peptide substrate, derived from the sequence of APP and containing a suitable fluorophore-quencher pair, is added to a final concentration of 1 to 5 µM, and the increase in fluorescence is recorded at a suitable excitation / emission wavelength in a microplate spectro-fluorimeter for 5 to 30 minutes in 1-minute intervals. IC₅₀ values are calculated from percentage of inhibition of BACE-1 activity as a function of the test compound concentration.

### Test 2: Inhibition of human BACE-2

Recombinant BACE-2 (extracellular domain, expressed in baculovirus and purified using standard methods) at 0.1 to 10 nM concentrations is incubated with the test compound at various concentrations for 1 hour at room temperature in 10 to 100 mM acetate buffer, pH 4.5, containing 0.1 % CHAPS. Synthetic peptide substrate, derived from the sequence of APP and containing a suitable fluorophore-quencher pair, is added to a final concentration of 1 to 5 µM, and the increase in fluorescence is recorded at a suitable excitation / emission wavelength in a microplate spectro-fluorimeter for 5 to 30 minutes in 1-minute intervals. IC₅₀ values are calculated from percentage of inhibition of BACE-2 activity as a function of the test compound concentration.

### Test 3: Inhibition of human cathepsin D

Recombinant cathepsin D (expressed as procathepsin D in baculovirus, purified using standard methods and activated by incubation in sodium formate buffer pH 3.7) is incubated with the test compound at various concentrations for 1 hour at room temperature in sodium formate or sodium acetate buffer at a suitable pH within the range of pH 3.0 to 5.0. Synthetic peptide substrate Mca-Gly-Lys-Pro-Ile-Leu-Phe-Phe-Arg-Leu-Lys(DNP)-D-Arg-NH₂ is added to a final concentration of 1 to 5 µM, and the increase in fluorescence is recorded at excitation of 325 nm and emission at 400 nm in a microplate spectro-fluorimeter for 5 to 30 minutes in 1-minute intervals. IC₅₀ values are calculated from the percentage of inhibition of cathepsin D-activity as a function of the test compound concentration.

### Test 4: Inhibition of cellular release of amyloid peptide 1-40

Chinese hamster ovary cells are transfected with the gene for amyloid precursor protein. The cells are plated at a density of 8000 cells/well into 96-well microtiter plates and cultivated for 24 hours in DMEM cell culture medium containing 10 % FCS. The test compound is added to the cells at various concentrations, and the cells are cultivated for 24 hours in the presence of the test compound. The supernatants are collected, and the concentration of amyloid peptide 1-40 is determined using state of the art immunoassay techniques, for example sandwich ELISA, homogenous time-resolved fluorescence (HTRF) immunoassay, or electrochemiluminescence immunoassay. The potency of the compound is calculated from the percentage of inhibition of amyloid peptide release as a function of the test compound concentration.

Agents of the invention were tested in at least one of the above-described tests. Specific activities of agents of the invention are described in Example 186.

As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

The term "a therapeutically effective amount" of a compound of the present invention refers to an amount of the compound of the present invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound of the present invention that, when administered to a subject, is effective to (1) at least partially alleviating, inhibiting, preventing and/or ameliorating a condition, or a disorder or a disease (i) mediated by BACE-1 or (ii) associated with BACE-1 activity, or (iii) characterized by activity (normal or abnormal) of BACE-1; or (2) reducing or inhibiting the activity of BACE-1. In another non-limiting embodiment, the term "a therapeutically effective amount" refers to the amount of the compound of the present invention that, when administered to a cell, or a tissue, or a non-cellular biological material, or a medium, is effective to at least partially reduce or inhibit the activity of BACE-1. The meaning of the term "a therapeutically effective amount" as illustrated in the above embodiments for BACE-1 also applies by the same means to any other relevant proteins/peptides/enzymes, such as BACE-2, or cathepsin D.

As used herein, the term "subject" refers to an animal. Typically the animal is a mammal. A subject also refers to for example, primates (*e.g*., humans, male or female), cows, sheep, goats, horses, dogs, cats, rabbits, rats, mice, fish, birds and the like. In certain embodiments, the subject is a primate. In yet other embodiments, the subject is a human.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "threat", "treating" or "treatment" refers to modulating the disease or disorder, either physically, (*e.g.*, stabilization of a discernible symptom), physiologically, (*e.g*., stabilization of a physical parameter), or both. In yet another embodiment, "treat", "treating" or "treatment" refers to preventing or delaying the onset or development or progression of the disease or disorder.

As used herein, a subject is "in need of" a treatment if such subject would benefit biologically, medically or in quality of life from such treatment.

As used herein, the term an "agent" of the invention is used interchangeably with the term a "compound" of the invention and has no difference in meaning therefrom.

As used herein, the term "a," "an," "the" and similar terms used in the context of the present invention (especially in the context of the claims) are to be construed to cover both the singular and plural unless otherwise indicated herein or clearly contradicted by the context. The use of any and all examples, or exemplary language (*e.g*. "such as") provided herein is intended merely to better illuminate the invention and does not pose a limitation on the scope of the invention otherwise claimed.

Due to their inhibiting properties towards proteases, agents of the invention may be useful in the treatment or prevention of a variety of disabilitating psychiatric, psychotic, neurological or vascular states, such as a condition, disease or disorder of the vascular system or of the nervous system, in which beta-amyloid generation or aggregation plays a role, or, based on the inhibition of BACE-2 (beta-site APP-cleaving enzyme 2) or cathepsin D, which are close homologues of the pepsin-type aspartyl proteases and beta-secretase, and the correlation of the BACE-2 or cathepsin D expression with a more tumorigenic or metastatic potential of tumor cells, as anti-cancer medicaments, such as in the suppression of the metastasis process associated with tumor cells. The said condition, disease or disorder of the vascular system or of the nervous system is exemplified by, and includes, without limitation, an anxiety disorder, such as panic disorder with or without agoraphobia, agoraphobia without history of panic disorder, an animal or other specific phobia, including a social phobia, social anxiety disorder, anxiety, obsessive-compulsive disorder, a stress disorder, including post-traumatic or acute stress disorder, or a generalized or substance-induced anxiety disorder; a neurosis; seizures; epilepsy, especially partial seizures, simple, complex or partial seizures evolving to secondarily generalized seizures or generalized seizures [absence (typical or atypical), myoclonic, clonic, tonic, tonic-clonic or atonic seizures]; convulsions; migraine; an affective disorder, including a depressive or bipolar disorder, e. g. single-episode or recurrent major depressive disorder, major depression, a dysthymic disorder, dysthymia, depressive disorder NOS, bipolar I or bipolar II manic disorder or cyclothymic disorder; a psychotic disorder, including schizophrenia or depression; neurodegeneration, e. g. neurodegeneration arising from cerebral ischemia; an acute, traumatic or chronic degenerative process of the nervous system, such as Parkinson's disease, Down's syndrome, dementia, e. g. senile dementia, dementia with Lewy bodies or a fronto-temporal dementia, a cognitive disorder, cognitive impairment, e. g. mild cognitive impairment, memory impairment, an amyloid neuropathy, a peripheral neuropathy, Alzheimer's disease, Gerstmann-Straeussler-Scheinker syndrome, Niemann-Pick disease, e. g. Niemann-Pick type C disease, brain inflammation, a brain, spinal cord or nerve injury, e. g. traumatic brain injury (TBI), a nerve trauma or a brain trauma, vascular amyloidosis, cerebral haemorrhage with amyloidosis, Huntington's chorea, amyotrophic lateral sclerosis, multiple sclerosis or fragile X syndrome; scrapie; cerebral amyloid angiopathy; an encephalopathy, e. g. transmissible spongiform encephalopathy; stroke; an attention disorder, e. g. attention deficit hyperactivity disorder; Tourette's syndrome; a speech disorder, including stuttering; a disorder of the circadian rhythm, e. g. in subjects suffering from the effects of jet lag or shift work; pain; nociception; itch; emesis, including acute, delayed or anticipatory emesis, such as emesis induced by chemotherapy or radiation, motion sickness, or post-operative nausea or vomiting; an eating disorder, including anorexia nervosa or bulimia nervosa; premenstrual syndrome; a muscle spasm or spasticity, e. g. in paraplegic patients; a hearing disorder, e. g. tinnitus or age-related hearing impairment; urinary incontinence; glaucoma; inclusion-body myositis; or a substance-related disorder, including substance abuse or dependency, including a substance, such as alcohol, withdrawal disorder. Agents of the invention may also be useful in enhancing cognition, e. g. in a subject suffering from a dementing condition, such as Alzheimer's disease; as pre-medication prior to anaesthesia or a minor medical intervention, such as endoscopy, including gastric endoscopy; or as ligands, e. g. radioligands or positron emission tomography (PET) ligands.

For the above-mentioned indications, the appropriate dosage will vary depending on, for example, the compound employed as active pharmaceutical ingredient, the host, the mode of administration, the nature and severity of the condition, disease or disorder or the effect desired. However, in general, satisfactory results in animals may be obtained at a daily dosage of from about 0.1 to about 100, preferably from about 1 to about 50, mg/kg of animal body weight. In larger mammals, for example humans, an indicated daily dosage is in the range of from about 0.5 to about 2000, preferably from about 10 to about 370, or from about 2 to about 200, mg of an agent of the invention conveniently administered, for example, in divided doses up to four times a day or in sustained release form. For the compound 5-cyano-3-methyl-pyridine-2-carboxylic acid [3-((R)-5-amino-3-difluoromethyl-3,6-dihydro-2H-[1,4]oxazin-3-yl)-4-fluoro-phenyl]-amide, a predicted daily dosage is in the range of 10 to 370 mg (total dose per day for a 70 kg person).

An agent of the invention may be administered by any conventional route, in particular enterally, preferably orally, e. g. in the form of a tablet or capsule, or parenterally, e. g. in the form of an injectable solution or suspension.

In a further aspect, the invention relates to a pharmaceutical composition comprising an agent of the invention as active pharmaceutical ingredient in association with at least one pharmaceutically acceptable carrier or diluent and optionally in association with other auxiliary substances, such as inhibitors of cytochrome P450 enzymes, agents preventing the degradation of active pharmaceutical ingredients by cytochrome P450, agents improving or enhancing the pharmacokinetics of active pharmaceutical ingredients, agents improving or enhancing the bioavailability of active pharmaceutical ingredients, and so on, e. g. grapefruit juice, ketoconazole or, preferably, ritonavir. Such a composition may be manufactured in conventional manner, e. g. by mixing its components. Unit dosage forms contain, e. g., from about 0.1 to about 1000, preferably from about 1 to about 500, mg of an agent of the invention.

For example, for preclinical animal studies a compound of the invention, such as 5-cyano-pyridine-2-carboxylic acid [3-((R)-5-amino-3-difluoromethyl-3,6-dihydro-2H-[1,4]oxazin-3-yl)-4-fluoro-phenyl]-amide, could be formulated as a suspension in a 0.5% methylcellulose solution with 0.1 % Tween80.

In addition, the pharmaceutical compositions of the present invention can be made up in a solid form (including without limitation capsules, tablets, pills, granules, powders or suppositories), or in a liquid form (including without limitation solutions, suspensions or emulsions). The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifers and buffers, etc.

Typically, the pharmaceutical compositions are tablets or gelatin capsules comprising the active ingredient together with
a) diluents, *e.g*., lactose, dextrose, sucrose, mannitol, sorbitol, cellulose and/or glycine;
b) lubricants, *e.g*., silica, talcum, stearic acid, its magnesium or calcium salt and/or polyethyleneglycol; for tablets also
c) binders, *e.g*., magnesium aluminum silicate, starch paste, gelatin, tragacanth, methylcellulose, sodium carboxymethylcellulose and/or polyvinylpyrrolidone; if desired
d) disintegrants, *e.g*., starches, agar, alginic acid or its sodium salt, or effervescent mixtures; and/or
e) absorbents, colorants, flavors and sweeteners.

Tablets may be either film coated or enteric coated according to methods known in the art.

Suitable compositions for oral administration include an effective amount of a compound of the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Certain injectable compositions are aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, or contain about 1-50%, of the active ingredient.

Suitable compositions for transdermal application include an effective amount of a compound of the invention with a suitable carrier. Carriers suitable for transdermal delivery include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Suitable compositions for topical application, *e.g*., to the skin and eyes, include aqueous solutions, suspensions, ointments, creams, gels or sprayable formulations, *e.g*., for delivery by aerosol or the like. Such topical delivery systems will in particular be appropriate for dermal application, *e.g*., for the treatment of skin cancer, *e.g*., for prophylactic use in sun creams, lotions, sprays and the like. They are thus particularly suited for use in topical, including cosmetic, formulations well-known in the art. Such may contain solubilizers, stabilizers, tonicity enhancing agents, buffers and preservatives.

As used herein a topical application may also pertain to an inhalation or to an intranasal application. They may be conveniently delivered in the form of a dry powder (either alone, as a mixture, for example a dry blend with lactose, or a mixed component particle, for example with phospholipids) from a dry powder inhaler or an aerosol spray presentation from a pressurised container, pump, spray, atomizer or nebuliser, with or without the use of a suitable propellant.

The present invention further provides anhydrous pharmaceutical compositions and dosage forms comprising the compounds of the present invention as active ingredients, since water may facilitate the degradation of certain compounds.

Anhydrous pharmaceutical compositions and dosage forms of the invention can be prepared using anhydrous or low moisture containing ingredients and low moisture or low humidity conditions. An anhydrous pharmaceutical composition may be prepared and stored such that its anhydrous nature is maintained. Accordingly, anhydrous compositions are packaged using materials known to prevent exposure to water such that they can be included in suitable formulary kits. Examples of suitable packaging include, but are not limited to, hermetically sealed foils, plastics, unit dose containers (*e. g*., vials), blister packs, and strip packs.

The invention further provides pharmaceutical compositions and dosage forms that comprise one or more agents that reduce the rate by which the compound of the present invention as an active ingredient will decompose. Such agents, which are referred to herein as "stabilizers," include, but are not limited to, antioxidants such as ascorbic acid, pH buffers, or salt buffers, etc.

In accordance with the foregoing, in a further aspect, the invention relates to an agent of the invention for use as a medicament, e. g. for the treatment or prevention of a neurological or vascular condition, disease or disorder, in which beta-amyloid generation or aggregation plays a role, or for the suppression of the metastasis process associated with tumor cells. In a further embodiment, the invention relates to an agent of the invention for use in the treatment of a disease or disorder mediated by BACE-1, BACE-2 or cathepsin D activity. In one embodiment, the invention relates to an agent of the invention for use in the treatment of Alzheimer's Disease.

An agent of the invention can be administered as sole active pharmaceutical ingredient or as a combination with at least one other active pharmaceutical ingredient effective, e. g., in the treatment or prevention of a neurological or vascular condition, disease or disorder, in which beta-amyloid generation or aggregation plays a role, or in the suppression of the metastasis process associated with tumor cells. Such a pharmaceutical combination may be in the form of a unit dosage form, which unit dosage form comprises a predetermined quantity of each of the at least two active components in association with at least one pharmaceutically acceptable carrier or diluent. Alternatively, the pharmaceutical combination may be in the form of a package comprising the at least two active components separately, e. g. a pack or dispenser-device adapted for the concomitant or separate administration of the at least two active components, in which these active components are separately arranged. In a further aspect, the invention relates to such pharmaceutical combinations.

In the combination therapies, the agent of the invention and the other therapeutic agent may be manufactured and/or formulated by the same or different manufacturers. Moreover, the compound of the invention and the other therapeutic may be brought together into a combination therapy: (i) prior to release of the combination product to physicians (*e.g*. in the case of a kit comprising the compound of the invention and the other therapeutic agent); (ii) by the physician themselves (or under the guidance of the physician) shortly before administration; (iii) in the patient themselves, *e.g*. during sequential administration of the compound of the invention and the other therapeutic agent.

The following Examples illustrate the invention.

### Examples

### Abbreviations

- ACN: acetonitrile
- AcOH: acetic acid
- Boc: tert-butoxycarbonyl
- Boc₂O: tert-butyl dicarbonate
- t-Bu: tert-butyl
- nBuLi: n-butyllithium
- t-BuOH: tert-butanol
- DAST: diethylaminosulfurtrifluoride (Et₂N)₂SF₃
- DCM: dichloromethane
- DIAD: diisopropyl azodicarboxylate
- DIPEA: diisopropylethylamine
- DMAP: 4-dimethylaminopyridine
- DMF: dimethylformamide
- DMSO: dimethylsulfoxide
- DPPF: 1,1'-bis(diphenylphosphino)ferrocene
- ee: enantiomeric excess
- EDC: 1-(3-dimethylaminopropyl)-3-ethylcarbodiimide hydrochloride
- eq: equivalent(s)
- Et₃N: triethylamine
- Et₂O: diethylether
- EtOAc: ethyl acetate
- EtOH: ethanol
- h: hour(s)
- Hex: hexane
- HOAt: 1-hydroxy-7-aza-benztriazole
- HOBT: hydroxy-benztriazole
- HPLC: high performance liquid chromatography
- IPAc: isopropyl acetate
- LCMS: liquid chromatography with mass spectrometry
- LDA: lithium diisopropylamide
- MeOH: methanol
- min: minute(s)
- MS: mass spectrometry
- NMR: nuclear magnetic resonance spectrometry
- NP: normal phase
- PE: petrolether
- PPh3: triphenylphosphine
- R_{f}: retention factor (TLC)
- RP: reverse phase
- Rt: retention time
- rt: room temperature
- SMB: simulated moving bed
- TBME: tert-butyl-methyl-ether
- TFA: trifluoroacetic acid
- THF: tetrahydrofuran
- TLC: thin layer chromatography
- UPLC: ultra performance liquid chromatography

### General chromatography information

### HPLC method H1 (Rt_{H1}):

| | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA; B) ACN + 0.05 Vol.-% TFA |
| HPLC-gradient: | 30 - 100 % B in 3.25 min, flow = 0.7 ml / min |

### HPLC method H2 (Rt_{H2}):

| | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA; B) ACN + 0.05 Vol.-% TFA |
| HPLC-gradient: | 0 - 100 % B in 3.25 min, flow = 0.7 ml / min |

### LCMS method H3 (Rt_{H3}):

| | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA, B) ACN + 0.05 Vol.-% TFA |
| HPLC-gradient: | 10 - 100 % B in 3.25 min, flow = 0.7 ml / min |

### LCMS method H4 Rt_{H4}):

| | |
|---|---|
| HPLC-column dimensions: | 3.0 x 30 mm |
| HPLC-column type: | Zorbax SB-C8, 1.8 µm |
| HPLC-eluent: | A) water + 0.05 Vol.-% TFA, B) ACN + 0.05 Vol.-% TFA |
| HPLC-gradient: | 10 - 95 % B in 2.00 min, 95 % B 2.00 min, |
| | flow = 0.7 ml / min |

### UPLC method H5 (Rt_{H5}):

| | |
|---|---|
| HPLC-column dimensions: | 2.1 x 50 mm |
| HPLC-column type: | Acquity UPLC HSS T3 C18, 1.7 µm |
| HPLC-eluent: | A) water + 0.1 Vol.-% TFA, B) ACN + 0.1 Vol.-% TFA |
| HPLC-gradient: | 5 - 100 % B in 1.5 min, flow = 1.0 ml / min |

### LCMS method H6 (Rt_{H6}):

| | |
|---|---|
| HPLC-column dimensions: | 3.0 x 30 mm |
| HPLC-column type: | Zorbax SB-C18, 1.81 µm |
| HPLC-eluent: | A) water + 0.05 Vol.-% TFA; B) ACN + 0.05 Vol.-% TFA |
| HPLC-gradient: | 40 - 100 % B in 3.25 min, flow = 0.7 ml / min |

### LCMS method H7 (Rt_{H7}):

| | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA; B) ACN + 0.05 Vol.-% TFA |
| HPLC-gradient: | 50 - 100 % B in 3.25 min, flow = 0.7 ml / min |

### LCMS method H8 (Rt_{H8}):

| | |
|---|---|
| HPLC-column dimensions: | 4.0 x 20 mm |
| HPLC-column type: | Mercury MS Synergi, 2 µm |
| HPLC-eluent: | A) water + 0.1 Vol.-% formic acid, B) ACN |
| HPLC-gradient: | 0.5 min 30% B, 30-95% B in 1 min, 0.9 min 95% B, flow = 2.0 ml / min |
| HPLC-column temperature: | 30 °C |

### LCMS method H9 (Rt_{H9}):

| | |
|---|---|
| HPLC-column dimensions: | 4.0 x 20 mm |
| HPLC-column type: | Mercury MS Synergi, 2 µm |
| HPLC-eluent: | A) water + 0.1 Vol.-% formic acid, B) ACN |
| HPLC-gradient: | 0.5 min 70% B, 70-100% B in 1 min, 0.6 min 70% B, flow = 2.0 ml / min |
| HPLC-column temperature: | 30 °C |

### UPLC method H10 (Rt_{H10}):

| | |
|---|---|
| HPLC-column dimensions: | 2.1 x 50 mm |
| HPLC-column type: | Acquity UPLC HSS T3, 1.8 µm |
| HPLC-eluent: | A) water + 0.05 Vol.-% formic acid + 3.75 mM ammonium acetate B) ACN + 0.04 Vol.-% formic acid |
| HPLC-gradient: | 2 - 98 % B in 1.7 min, 98% B 0.45 min, flow = 1.2 ml / min |

### LCMS method H11 (Rt_{H11}):

| | |
|---|---|
| HPLC-column dimensions: | 2.1 x 30 mm |
| HPLC-column type: | Ascentis Express C18, 2.8 µm |
| HPLC-eluent: | A) water + 0.05 Vol.-% formic acid + 3.75 mM ammonium acetate, B) ACN + 0.04 Vol.-% formic acid |
| HPLC-gradient: | 2 - 98 % B in 1.4 min, 0.75 min 98% B, flow = 1.2 ml / min |
| HPLC-column temperature: | 50 °C |

### Reference Example 42: 5-Bromo-pyridine-2-carboxylic acid [3-(5-amino-3-difluoromethyl-3,6-dihydro-2H-[1,4]oxazin-3-yl)-4-fluoro-phenyl]-amide hydrochloride

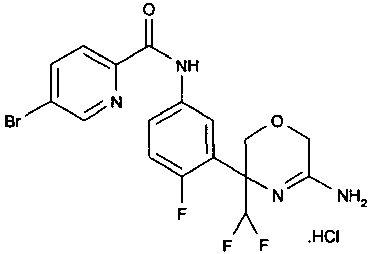

### a) 1-(5-Bromo-2-fluoro-phenyl)-ethanone

A solution of 17.78 ml (126 mmol) diisopropyl amine in 375 ml THF was cooled to -78°C. A 1.6 M solution of BuLi in hexanes (79 ml, 126 mmol) was added drop wise. After 15 minutes 20 g of 4-bromo-1-fluoro benzene (114 mmol) was added dropwise while keeping the temperature below -60°C. After stirring for 2.5 h at -70°C 13.22 ml ethyl difluoro acetate were added. The mixture was warmed to -40°C and then quenched by pouring the mixture onto 1M HCl. The mixture was extracted with ligroine, dried with MgSO₄.H₂O, concentrated and purified by column chromatography (silica gel; hexane/5-15% TBME) to give the desired product as a yellow liquid. ¹H-NMR (CDC₃, 360 MHz): 8.09 (dd, 1 H), 7.82-7.77 (m, 1H), 7.17 (t, 1 H), 6.45 (t, 1 H, CHF₂)

### b) 1-(5-Bromo-2-fluoro-phenyl)-1-difluoromethyl-allyl]-carbamic acid tert-butyl ester

A mixture of 16 g (63.2 mmol) 1-(5-bromo-2-fluoro-phenyl)-ethanone and 26.3 g (69.6 mmol) N-tert-butyloxycarbonyl-triphenyliminophosphorane were heated at 90°C in toluene for 18 h. The mixture was triturated with hexane and filtered to remove triphenyl phosphine oxide. The filtrate was purified by chromatography on silica gel (hexane/1-5% TBME) to give 11.37 g (32.3 mmol) of the desired product as a slightly impure yellow oil. TLC: Rf (Hexane / EtOAc 6:1) = 0.65.
The product was dissolved in 100 ml THF and cooled to -78°C. Vinylmagnesium bromide (48 ml of a 1 M solution in THF) was added dropwise, while the reaction temperature was not allowed to exceed -60°C. The mixture was stirred at -70°C for 1 h before it was allowed to warm to 0°C. The reaction was quenched with 10% aqueous ammonium chloride and extracted with TBME. The organic layer was washed with brine, treated with activated charcoal and MgSO4.H2O and filtered over celite. The filtrated was concentrated and crystallized from hexane to give the desired product as colorless crystals.
HPLC: Rt_{H1}= 3.575 min; ESIMS [M+Na]⁺ =402/404(1Br);
¹H-NMR (CDCl₃, 360 MHz): 7.57 (dd, 1 H), 7.51-7.45 (m, 1H), 7.00 (dd, 1H), 6.49 (t, 1H, CHF₂), 6.21 (dd, 1 H), 5.59 (d, 1 H), 5.40 (dd, 1 H), 5.25 (br, 1H), 1.40 (br s, 9H).

### c) [1-(5-Bromo-2-fluoro-phenyl)-2,2-difluoro-1-hydroxymethyl-ethyl]-carbamic acid tert-butyl ester

A suspension of 10.99 g (28.9 mmol) 1-(5-bromo-2-fluoro-phenyl)-1-difluoromethyl-allyl]-carbamic acid tert-butyl ester and 3.84 g (43.4 mmol) sodium hydrogen carbonate in 200 ml DCM and 80 ml MeOH was cooled to -78°C. A mixture of O₃ in oxygen gas was introduced till the blue color persisted. The excess ozone was removed by bubbling through oxygen gas for 10 minutes. NaBH4 (2.187 g, 57.8 mmol) was added as a solid in three portions. The mixture was stirred 10 min at -78°C and then allowed to warm to 0°C. After 30 min the mixture was poured onto ice-cold 1 N HCl and extracted with TBME. The organic phase was washed with 1N HCl, brine, dried with MgSO4.H2O and evaporated. The crude product was crystallized from hexane to give the desired product as colorless crystals.
TLC: Rf (Hexane / EtOAc 4:1) = 0.29.
HPLC: Rt_{H1}= 3.000 min; ESIMS [M+Na]⁺ =406/408(1Br);
¹H-NMR (DMSO-d6, 360 MHz): 7.60-7.49 (m, 2H), 7.42 (br s, 1 H), 7.180 (dd, 1H), 6.49 (t, 1 H, CHF₂), 5.27 (br s, 1 H), 3.90 (br s, 2H), 1.35 (br s, 9H).

### d) N-[1-(5-Bromo-2-fluoro-phenyl)-2,2-difluoro-1-hydroxymethyl-ethyl]-2-chloro-acetamide

A suspension of 10.22 g (26.6 mmol) [1-(5-bromo-2-fluoro-phenyl)-2,2-difluoro-1-hydroxymethyl-ethyl]-carbamic acid tert-butyl ester in 133 ml 4N HCl in dioxane was stirred for two h at rt. The mixture was evaporated to give the hydrochloride salt of 2-amino-2-(5-bromo-2-fluoro-phenyl)-3,3-difluoro-propan-1-ol.
HPLC: Rt_{H3}= 2.550 min; ESIMS [M+H]⁺ =284,286(1Br);
The crude product was taken up in 63 ml DCM and 63 ml 10% aqueous soda and stirred vigorously with ice-cooling. A solution of 3.34 ml (42 mmol) chloroacetyl chloride in 10 ml DCM was added dropwise. The ice bath was taken away and stirring was continued for 1 h. The mixture was diluted with TBME and water. The organic phase was dried with MgSO4.H2O and purified via chromatography on silica gel (hexane/25-33% EtOAc) to give the desired product as a slightly impure resin.
HPLC: Rt_{H3}= 3.336 min; ESIMS [M+H]⁺ =360/362/364 (1Br, 1Cl);
¹H-NMR (DMSO-d6, 360 MHz): 8.78 (s, 1 H), 7.62-7.53 (m, 2H), 7.19 (dd, 1 H), 6.53 (t, 1H, CHF₂), 5.43 (t, 1 H), 4.27-4.02 (m, 4H).

### e) 5-(5-Bromo-2-fluoro-phenyl)-5-difluoromethyl-morpholin-3-one

A solution of 9.59 g (26.2 mmol) N-[1-(5-bromo-2-fluoro-phenyl)-2,2-difluoro-1-hydroxymethyl-ethyl]-2-chloro-acetamide in 134 ml t-butanol was treated with 3.58 g KOtBu. The mixture was heated at reflux for 3h. After cooling down the mixture was diluted with EtOAc and 1 N HCl. The organic phase was washed with brine, dried with MgSO4.H2O, filtered and evaporated. The product was obtained as colorless crystal (TBME/hexane). TLC: Rf (Hexane / EtOAc 2:1) = 0.29.
HPLC: Rt_{H3}= 2.950 min; ESIMS [M+H]⁺ =324/326(1 Br);
¹H-NMR (CDCl3, 360 MHz): 7.61-7.55 (m, 2H), 7.09 (dd, 1 H), 6.80 (br, 1H), 6.35 (t, 1H, CHF2), 4.37-4.17 (m, 4H).

### f) 5-(5-Bromo-2-fluoro-phenyl)-5-difluoromethyl-morpholine-3-thione

A mixture of 7.34 g (22.65 mmol) 5-(5-bromo-2-fluoro-phenyl)-5-difluoromethyl-morpholin-3-one and 5.19 g (12.46 mmol) Lawesson's reagent in 73 ml of THF was refluxed for 1 h. The mixture was concentrated and crystallized from DCM/hexane and recrystallized from EtOH to yield the desired product as colorless crystals.
HPLC: Rt_{H3}= 3.370 min; ESIMS [M+H]⁺ =340/342(1 Br);
¹H-NMR (DMSO-d6, 360 MHz): 11.40 (s, 1 H), 7.77-7.70 (m, 1H), 7.63 (dd, 1 H), 7.37 (dd, 1H), 6.35 (t, 1H, CHF2), 4.50 (d, 1 H), 4.44 (d, 1H), 4.29 (d, 1H), 4.10 (d, 1 H).

### g) 5-(5-Bromo-2-fluoro-phenyl)-5-difluoromethyl-5,6-dihydro-2H-[1,4]oxazin-3-ylamine

A solution of 6.14 g (18.05 mmol) 5-(5-bromo-2-fluoro-phenyl)-5-difluoromethyl-morpholine-3-thione in 77 ml 7M NH3/MeOH was stirred at rt for 6h. The mixture was evaporated and purified chromatographed on silica gel (DCM/1-5% MeOH followed by DCM/MeOH/aqueous NH3 95:4.5:0.5) to give the desired product as yellowish resin.
HPLC: Rt_{H3}= 2.477 min; ESIMS [M+H]⁺ =323/325(1Br);
¹H-NMR (DMSO-d6, 360 MHz): 7.99 (dd, 1H), 7.62-7.56 (m, 1 H), 7.22 (dd, 1H), 6.31 (br, 2H), 6.12 (t, 1H, CHF2), 4.25 (d, 1 H), 4.05 (d, 1H), 3.94 (d, 1 H), 3.75 (d, 1 H).

### h) [5-(5-Bromo-2-fluoro-phenyl)-5-difluoromethyl-5,6-dihydro-2H-[1,4]oxazin-3-yl]-carbamic acid tert-butyl ester

To an ice-cold solution of 6.38 g (19.75 mmol) 5-(5-bromo-2-fluoro-phenyl)-5-difluoromethyl-5,6-dihydro-2H-[1,4]oxazin-3-ylamine in 100 ml THF were added 5.60 g (25.67 mmol) Boc2O and 5.17 ml (29.6 mmol) DIPEA. The mixture was stirred for 4h at rt. Then the mixture was diluted with TBME and washed with 5% aqueous NaHCO3. The organic phase was dried with MgSO4.H2O filtered and concentrated. Purification by chromatography on silica gel (hexane/ 5-20% EtOAc) gave the desired product as a colorless solid.
TLC: Rf (Hexane / EtOAc 9:1) = 0.27.
HPLC: Rt_{H1}= 3.299 min; ESIMS [M+H]⁺ =423/425(1Br);
¹H-NMR (CDCl3, 360 MHz): 7.81 (dd, 1 H), 7.50-7.44 (m, 1 H), 7.00 (dd, 1 H), 6.12 (t, 1H, CHF2), 4.83 (d, 1 H), 4.60 (d, 1 H), 4.37 (dd, 1 H), 3.94 (d, 1H), 1.52 (s, 9H).

### i) [5-(5-Azido-2-fluoro-phenyl)-5-difluoromethyl-5,6-dihydro-2H-[1,4]oxazin-3-yl]-carbamic acid tert-butyl ester

To a solution of 7.27g (17.18 mmol) [5-(5-bromo-2-fluoro-phenyl)-5-difluoromethyl-5,6-dihydro-2H-[1,4]oxazin-3-yl]-carbamic acid tert-butyl ester and 2.443 g (17.18 mmol) trans-N,N'-dimethylcyclohexane-1,2-diamine in 237 ml EtOH was added a solution of 8.93 g (137 mmol) sodium azide and 1.361 g (6.87 mmol) sodium-ascorbate in 102 ml water. The mixture was degassed and brought under nitrogen atmosphere. Cul (1.309 g, 6.87 mmol) was added and the mixture was heated at 70°C. The initially formed suspension turned into a homogeneous blue solution. The mixture was cooled to rt, diluted with water and TBME. The organic phase was washed with brine and dried with MgSO4.H2O. The crude product was purified by chromatography on silica gel (hexane / 5-8% TBME) to give the desired product as a colorless solid.
HPLC: Rt_{H1}= 3.173 min; ESIMS [M+H]⁺=386;
¹H-NMR (CDCl3, 360 MHz, signals broadened due to rotamers): 7.39-7.44 (m, 1 H), 7.15-7.06 (m, 1 H), 7.05-6.98 (m, 1 H), 6.14 (t, 1H, CHF2), 4.80 (d, 1 H), 4.60 (d, 1 H), 4.39 (d, 1 H), 3.97 (d, 1H), 1.52 (s, 9H).

### j) [5-(5-Amino-2-fluoro-phenyl)-5-difluoromethyl-5,6-dihydro-2H-[1,4]oxazin-3-yl]-carbamic acid tert-butyl ester

A solution of 4.89 g (12.69 mmol) [5-(5-azido-2-fluoro-phenyl)-5-difluoromethyl-5,6-dihydro-2H-[1,4]oxazin-3-yl]-carbamic acid tert-butyl ester in 64 ml EtOH and 17 ml THF was treated with 1.1 g 10% Pd-C and stirred under an atmosphere of hydrogen until the starting material had been consumed. The mixture was diluted with DCM and filtered over celite. The product was purified by chromatography on silica gel (hexane / 25-50% EtOAc) to give the desired product as colorless foam.
HPLC: Rt_{H3}= 2.778 min; ESIMS [M+H]⁺ =360;
¹H-NMR (CDCl3, 360 MHz, spectrum not interpretable due to rotamers): 7.1-6.1 (m, -4H), 5.0-4.9 (m, -4H), 1.52 (br s, 9H).

### k) (5-{5-[(5-Bromo-pyridine-2-carbonyl)-amino]-2-fluoro-phenyl}-5-difluoromethyl-5,6-dihydro-2H-[1,4]oxazin-3-yl)-carbamic acid tert-butyl ester

To a solution of 325 mg (0.952 mmol) [5-(5-amino-2-fluoro-phenyl)-5-difluoromethyl-5,6-dihydro-2H-[1,4]oxazin-3-yl]-carbamic acid tert-butyl ester, 212 mg (1.047 mmol) 5-bromo-pyridine-2-carboxylic acid, 168 mg (1.238 mmol) HOAt and 0.34 ml (2.38 mmol) Et₃N in 5 ml DCM were added 237 mg (1.24 mmol) EDC.HCl. The mixture was stirred overnight. The mixture was diluted with EtOAc and washed with water, 1 N HCl, brine and 5% aqueous NaHCO₃. The organic phase was dried with MgSO₄.H2O, filtered and purified by chromatography on silica gel (hexane / 14-18% EtOAc) to give the desired product as colorless foam.
TLC: Rf (Hexane / EtOAc 3:1) = 0.35.
HPLC: Rt_{H1}= 3.127 min; ESIMS [M+H]⁺ =525/527(1 Br);
¹H-NMR (CDCl3, 360 MHz): 9.90 (br s, 1 H), 8.72 (d, 1 H), 8.23 (d, 1H), 8.09 (dd, 1H), 7.94-7.86 (m, 2H), 7.47 (t, 1H), 7.38-7.28 (m, 3H), 5.92 (t, 1H, CHF2), 4.87 (d, 1 H), 4.67 (d, 1 H), 4.6-4.45 (br, 1 H), 4.34 (d, 1 H), 4.00 (d, 1 H), 1.56 (br s, 9H).

### I) 5-Bromo-pyridine-2-carboxylic acid [3-(5-amino-3-difluoromethyl-3,6-dihydro-2H-[1,4]oxazin-3-yl)-4-fluoro-phenyl]-amide

A solution of 100 mg (0.184 mmol) (5-{5-[(5-bromo-pyridine-2-carbonyl)-amino]-2-fluoro-phenyl}-5-difluoromethyl-5,6-dihydro-2H-[1,4]oxazin-3-yl)-carbamic acid tert-butyl ester in 1.4 ml 4N HCl in dioxane was stirred at 50°C. After 15 min 0.3 ml 3N HCl in MeOH were added and the now homogeneous solution was stirred for 3 h. The mixture was concentrated and crystallized from EtOH/TBME to yield the title compound.
HPLC: Rt_{H3}= 2.857 min; ESIMS [M+H]⁺ =443/445;
¹H -NMR (600 MHz, DMSO-d6): δ 10.93 (s, 1 H), 9.78 (s, 1H), 8.89 (s, 1 H), 8.77 (s, 1 H), 8.35 (d, 1H), 8.11-8-06 (m, 3H), 7.39 (t, 1H), 6.79 (t, 1H, CHF2), 4.70 (d, 1 H), 4.64 (d, 1H), 4.34 (d, 1 H), 4.17 (d, 1H).

### Reference Example 71: 5-Bromo-pyrimidine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride

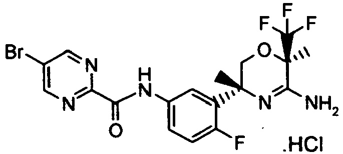

### a) 2-(5-Bromo-2-fluoro-phenyl)-propan-2-ol

To a solution of diisopropyl amine (57.3 ml, 402 mmol) in THF (500 ml) was added under argon a 1.6 M solution of nBuLi in hexane (260 ml, 416 mmol) below -50 °C. After stirring for 30 min at -75 °C, 4-bromo-1-fluoro benzene (31.1 ml, 277 mmol) was added while keeping the temperature below -70 °C. After stirring for 2 h at -75 °C, acetone (41.2 ml, 554 mmol) was added below -65 °C and the reaction mixture was stirred for 1 h at -75 °C, warmed up to -50 °C and poured onto 10% aqueous NH₄Cl solution. The mixture was extracted with TBME, organic phases were washed with aqueous KHSO₄ solution, saturated NaHCO₃ solution and brine, dried over MgSO₄, filtered and concentrated. The crude product was crystallized from hexane to provide the title compound as white crystals: TLC (hexane-EtOAc 3:1): Rf =0.45; HPLC Rt_{H5}=1.045 min; ¹H NMR (360 MHz, CDCl₃): δ 7.74 (dd, 1H), 7.36 (m, 1 H), 6.93 (dd, 1 H), 2.04 (d, 1 H), 1.63 (s, 6H).

### b) 4-Bromo-1-fluoro-2-isopropenyl-benzene

To a solution of 2-(5-bromo-2-fluoro-phenyl)-propan-2-ol (119.7g, 498 mmol) in CH₂Cl₂ (50 ml) was added hydrochinone (2.74 g, 24.9 mmol) and 250 ml 85% H₃PO₄. The resulting reaction mixture was stirred for 3.5 h at 50 °C. The mixture was poured onto ice-water and extracted with CH₂Cl₂. The organic phases were washed with 2N aqueous NaOH and water, dried over MgSO₄, filtered and concentrated. The crude product was dissolved in hexane and filtered through a plough of silica gel to obtain after concentration at 600 mbar the title compound as a colorless oil: TLC (hexane): Rf =0.52; HPLC Rt_{H5}=1.416 min; ¹H NMR (360 MHz, CDCl₃): δ 7.43 (dd, 1 H), 7.37 (m, 1 H), 6.94 (dd, 1 H), 5.27 (d, 2H), 2.13 (s, 3H).

### c) (S)-2-(S-Bromo-2-fluoro-phenyl)-propane-1,2-diol

To a suspension of K₃Fe(CN)₆ (186 g, 561 mmol), K₂CO₃ (78 g, 561 mmol), (DHQ)₂-PHAL (1.311 g, 1.674 mmol) and K₂OsO₂(OH)₄ (0.378 g, 1 mmol) in t-BuOH-H₂O 1:1 (1600 ml) was added 4-bromo-1-fluoro-2-isopropenyl-benzene (36 g, 167 mmol) at 0 °C and the reaction mixture was stirred for 14 h at 0 °C. After careful addition of Na₂S₂O₅ (100 g) at 0-5 °C the reaction mixture was stirred for 1 h before extraction with EtOAc. Combined extracts were washed with 5% NaS₃O₃ solution and brine, dried over MgSO4, filtered and concentrated to give the title compound as a white solid: TLC (hexane-EtOAc 1:1): Rf =0.46; HPLC Rt_{H5}=0.767 min; ESIMS: 266, 268 [(M+NH₄)⁺]; ¹H NMR (360 MHz, CDCl₃): δ 7.71 (dd, 1 H), 7.27 (m, 1 H), 6.83 (dd, 1 H), 3.85 (d, 1 H), 3.62 (d, 1 H), 2.94 (s, 3H), 2.01 (s, 1 H), 1.43 (s, 3H).

### d) (S)-2-(5-Bromo-2-fluoro-phenyl)-2-methyl-oxirane

To a solution of (S)-2-(5-bromo-2-fluoro-phenyl)-propane-1,2-diol (37.35 g, 150 mmol) in CH₂Cl₂ (400 ml) was added under argon NEt₃ (41.8 ml, 300 mmol) and dropwise mesyl chloride (12.8 ml, 165 mmol) at 0-5 °C. After stirring for 0.5 h at 0-5 °C the reaction mixture was added to cold 1 N HCl and extracted with CH₂Cl₂. Combined extracts were washed with 1 N HCl, H₂O and saturated NaHCO₃ solution, dried over MgSO₄, filtered and concentrated. The crude mesylate was dissolved in TBME (500 ml) and 200 ml 2N aqueous NaOH and after stirring for 2 h at 25 °C the mixture was extracted with TBME. Combined extracts were washed with NaH₂PO₄ solution and brine, dried over MgSO₄, filtered and concentrated to provide the (S)-enantiomer as a colorless oil: 78% ee (Chiralpak AS-H 1218, hexane-EtOH 97:3, 0.4 mL/min); TLC (hexane-EtOAc 3:1): Rf =0.69; HPLC Rt_{H5}= 1.186 min; ¹H NMR (360 MHz, CDCl₃): δ 7.46 (dd, 1 H), 7.30 (m, 1 H), 6.83 (dd, 1H), 2.88 (d, 1 H), 2.72 (d, 1 H), 1.59 (s, 3H).

### e) (S)-1-Azido-2-(5-bromo-2-fluoro-phenyl)-propan-2-ol

To a solution of (S)-2-(5-bromo-2-fluoro-phenyl)-2-methyl-oxirane (51.85 g, 224 mmol) in EtOH (800 ml) was added NaN₃ (36.8 g, 531 mmol), NH₄Cl (60.6 g, 1122 mmol) and 18-crown-6 (59.8 g, 224 mmol) and the reaction mixture was heated at reflux for 6 h. The reaction mixture was filtered and concentrated to half of its volume. The residual oil was extracted with EtOAc. Combined extracts were washed with saturated NaHCO₃ solution and brine, dried over MgSO₄, filtered and concentrated to provide the title compound as a light yellow oil: TLC (hexane-EtOAc 1:1): Rf =0.70; HPLC Rt_{H3}= 1.115 min; ¹H NMR (360 MHz, CDCl₃): δ 7.72 (dd, 1 H), 7.32 (m, 1 H), 6.85 (dd, 1 H), 3.73 (d, 1 H), 3.51 (d, 1 H), 2.44 (s, 1 H), 1.50 (s, 3H).

### f) (S)-1-Amino-2-(5-bromo-2-fluoro-phenyl)-propan-2-ol

To a suspension of LiAlH₄ (4.65 g, 122 mmol) in THF (250 ml) was added under argon at 0-5 °C a solution of (S)-1-azido-2-(5-bromo-2-fluoro-phenyl)-propan-2-ol (33.4 g, 122 mmol) dissolved in THF (150 ml) over a period of 30 min. After stirring for 1 h at 0-5 °C, the reaction was quenched by careful addition of water (4.7 ml), 4 N NaOH (4.7 ml) and water (14.1 ml) and stirred again for 3 h at 25 °C. The white suspension was dried with MgSO₄, filtered and concentrated. The solidified product was re-crystallized from TBME-hexane to provide the title compound as beige crystals: 98% ee (Chiralpak AD-H hexane-EtOH 75-25 + 0.05% NEt₃); TLC (CH₂Cl₂-MeOH 10:1) Rf =0.10; HPLC Rt_{H5}= 0.558 min; ESIMS: 248, 250 [(M+H)⁺]; ¹H NMR (360 MHz, CDCl₃): δ 7.76 (dd, 1 H), 7.25 (m, 1 H), 6.82 (dd, 1 H), 4.16 (br s, 1 H), 3.19 (d, 1H), 2.72 (d, 1 H), 1.44 (s, 3H), 0.95 (br s, 2H).

### g) N-[(S)-2-(5-Bromo-2-fluoro-phenyl)-2-hydroxy-propyl]-2-nitro-benzenesulfonamide

To a solution of (S)-1-amino-2-(5-bromo-2-fluoro-phenyl)-propan-2-ol (34.7 g, 140 mmol) in THF (400 ml) was added 2-nitro-benzenesulfonyl chloride (34.9 g, 154 mmol) at 0-5 °C and afterwards 1 N aqueous NaOH over a period of 0.5 h. The reaction mixture was stirred for 2 h at 20 °C. The reaction mixture was diluted with TBME and washed with water and NaH₂PO₄ solution and brine, dried over MgSO₄, filtered and concentrated to provide the title compound after crystallization from TBME-hexane as beige crystals: TLC (toluene-EtOAc 3:1): Rf =0.51; HPLC Rt_{H5}= 1.118 min; ESIMS: 450, 452 [(M+NH₄)⁺]; ¹H NMR (360 MHz, CDCl₃): δ 7.98 (m, 1H), 7.81 (m, 1 H), 7.65 (m, 2H), 7.59 (dd, 1 H), 7.24 (m, 1 H), 6.79 (dd, 1 H), 5.60 (t, 1H), 4.16 (br s, 1 H), 3.55 (dd, 1 H), 3.44 (dd, 1 H), 2.51 (s, 1 H), 1.51 (s, 3H).

### h) (R)-2-(5-Bromo-2-fluoro-phenyl)-2-methyl-1-(2-nitro-benzenesulfonyl)-aziridine

To a solution of N-[(S)-2-(5-bromo-2-fluoro-phenyl)-2-hydroxy-propyl]-2-nitro-benzenesulfon-amide (20.8 g, 48 mmol) in CH₂Cl₂ (400 ml) was added PPh₃ (19.2 g, 72.4 mmol) at 0-5 °C and diethyl azodicarboxylate (11.6 ml, 72.4 mmol). The reaction mixture was stirred for 24 h at 25 °C and concentrated. The title compound was obtained after chromatographic purification over silica gel (hexane-EtOAc 20:1 to 2:1) as yellow crystals: TLC (toluene-EtOAc 3:1): Rf =0.69; HPLC Rt_{H5}= 1.308 min; ¹H NMR (360 MHz, CDCl₃): δ 8.31 (m, 1H), 7.28 (m, 3H), 7.60 (dd, 1 H), 7.42 (m, 1 H), 6.91 (dd, 1 H), 3.24 (s, 1 H), 2.81 (s, 1 H), 2.06 (s, 3H).

### i) (R)-2-[(R)-2-(5-Bromo-2-fluoro-phenyl)-2-(2-nitro-benzenesulfonylamino)-propoxy]-3,3,3-trifluoro-2-methyl-propionic acid ethyl ester

To a suspension of NaH (2.53 g 60% in mineral oil, 63 mmol) in DMF (160 ml) was added drop-wise under argon (R)-3,3,3-trifluoro-2-hydroxy-2-methyl-propionic acid ethyl ester (11.99 g, 63 mmol) and after stirring for 0.5 h at 20 °C (R)-2-(5-bromo-2-fluoro-phenyl)-2-methyl-1-(2-nitro-benzenesulfonyl)-aziridine (21.85 g, 52.6 mmol). The reaction was kept at 25 °C for 16 h. The mixture was added to cold aqueous 2N HCl and the product extracted with TBME. Combined organic layers were washed with saturated NaHCO₃ solution and brine, dried over MgSO₄, filtered and concentrated. The residual solid was re-crystallized from TBME-hexane to provide the title compound as yellow crystals: TLC (hexane-EtOAc 1:1): Rf =0.59; HPLC Rt_{H5}= 1.444 min; ESIMS: 618, 620 [(M+NH₄)⁺]; ¹H NMR (360 MHz, CDCl₃): δ 7.83 (dd, 1 H), 7.61 (m, 3H), 7.48 (dd, 1H), 7.27 (m, 1H), 6.73 (s, 1H), 6.60 (dd, 1H), 4.33 (m, 2H), 3.84 (s, 2H), 1.84 (s, 3H), 1.57 (s, 3H), 1.33 (t, 3H).

### j) (R)-2-[(R)-2-(5-Bromo-2-fluoro-pheyl)-2-(2-nitro-benzenesulfonylamino)-propoxy]-3,3,3-trifluoro-2-methyl-propionamide

A solution of (R)-2-[(R)-2-(5-bromo-2-fluoro-phenyl)-2-(2-nitro-benzenesulfonylamino)-propoxy]-3,3,3-trifluoro-2-methyl-propionic acid ethyl ester (26.6 g, 44.2 mmol) in 7N NH₃ in MeOH (75 ml) was stirred for 16 h at 50 °C. The solvent was removed under reduced pressure and the residual solid re-crystallized from Et₂O to give the title compound as yellow crystals: TLC (hexane-EtOAc 1:1): Rf =0.35; HPLC Rt_{H5}= 1.184 min; ESIMS: 589, 591 [(M+NH₄)⁺]; ¹H NMR (360 MHz, CDCl₃): δ 7.85 (d, 1H), 7.64 (m, 3H), 7.44 (d, 1H), 7.41 (dd, 1H), 7.26 (m, 1 H), 6.68 (br s, 1 H), 6.57 (dd, 1H), 6.19 (s, 1H), 5.54 (br s, 1H), 4.24 (d, 1 H), 3.93 (d, 1 H), 1.79 (s, 3H), 1.67 (s, 3H).

### k) N-[(R)-1-(5-Bromo-2-fluoro-phenyl)-2-((R)-1-cyano-2,2,2-trifluoro-1-methyl-ethoxy)-1-methyl-ethyl]-2-nitro-benzenesulfonamide

To a solution of (R)-2-[(R)-2-(5-bromo-2-fluoro-pheyl)-2-(2-nitro-benzenesulfonylamino)-propoxy]-3,3,3-trifluoro-2-methyl-propionamide (20.83 g, 35.6 mmol) in CH₂Cl₂ (300 ml) was added under argon NEt₃ (12.5 ml, 89 mmol) and at 0-5 °C trifluoroacetic anhydride (6.15 ml, 42.7 mmol). After stirring for 4 h at 25 °C the reaction mixture was added to a cold NaHCO₃ solution and the product was extracted with CH₂Cl₂. Combined extracts were washed with cold 0.1 N aqueous HCl, water and saturated NaHCO₃ solution, dried over MgSO₄, filtered and concentrated to provide the title compound as a yellow oil, which was used as such for the next step: TLC (hexane-EtOAc 1:1): Rf =0.73; HPLC Rt_{H5}= 1.364 min; ESIMS: 571, 573 [(M+NH₄)⁺]; ¹H NMR (360 MHz, CDCl₃): δ 7.89 (d, 1 H), 7.62 (ddd, 1 H), 7.57 (ddd, 1 H), 7.52 (m, 2H), 7.29 (m, 1H), 6.58 (dd, 1H), 6.19 (s, 1H), 4.17 (s, 2H), 1.81 (s, 3H), 1.72 (s, 3H).

### I) (2R,5R)-5-(5-Bromo-2-fluoro-phenyl)-2,5-dimethyl-2-trifluoromethyl-5,6-dihydro-2H-[1,4]oxazin-3-ylamine

To a solution of N-[(R)-1-(5-bromo-2-fluoro-phenyl)-2-((R)-1-cyano-2,2,2-trifluoro-1-methyl-ethoxy)-1-methyl-ethyl]-2-nitro-benzenesulfonamide (6.54 g,11.8 mmol) and N-acetylcysteine (2.4 g, 26.0 mmol) in MeOH (80 ml) was added K₂CO₃ (3.62 g, 26.0 mmol) and the reaction mixture was heated at 80 °C for 16 h. After removal of the solvent the residue was dissolved in water and extracted with EtOAc. Combined extracts were washed with saturated NaHCO₃ solution and brine, dried over MgSO₄, filtered and concentrated to provide the title compound after after chromatographic purification over silica gel (hexane-EtOAc 10:1 to 1:2 containing 0.03% NEt₃) as a yellow oil: TLC (hexane-EtOAc 1:1): Rf =0.58; HPLC Rt_{H5}= 0.843 min; ESIMS: 369, 371 [(M+H)⁺]; ¹H NMR (360 MHz, CDCl₃): δ 7.66 (dd, 1 H), 7.35 (m, 1H), 6.91 (dd, 1H), 3.97 (m, 2H), 1.53 (s, 3H), 1.49 (s, 3H).

### m) (2R,5R)-5-(2-Fluoro-phenyl)-2,5-dimethyl-2-trifluoromethyl-5,6-dihydro-2H-[1,4]oxazin-3-ylamine

A solution of (2R,5R)-5-(5-bromo-2-fluoro-phenyl)-2,5-dimethyl-2-trifluoromethyl-5,6-dihydro-2H-[1,4]oxazin-3-ylamine (1.66 g, 4.5 mmol) and sodium acetate (0.369 g,4.5 mmol) in MeOH (50 ml) was hydrogenated over 10% Pd-C for 6 h at 50 °C. The catalyst was filtered off over Celite and the filtrate was concentrated. The residue was dissolved in saturated NaHCO₃ solution and extracted with EtOAc. Combined extracts were washed with brine, dried over MgSO₄, filtered and concentrated to provide the title compound as a colorless oil: TLC (hexane-EtOAc 1:1): Rf =0.19; HPLC Rt_{H5}= 0.777 min; ESIMS: 291 [(M+H)⁺]; ¹H NMR (360 MHz, CDCl₃): δ 7.41 (dt, 1 H), 7.26 (m, 1 H), 7.11 (t, 1 H), 7.05 (dd, 1 H), 4.11 (dd, 1 H), 3.94 (dd, 1 H), 1.54 (s, 3H), 1.49 (s, 3H).

### n) (2R,5R)-5-(2-Fluoro-5-nitro-phenyl)-2,5-dimethyl-2-trifluoromethyl-5,6-dihydro-2H-[1,4]oxazin-3-ylamine

To a solution of (2R,5R)-5-(2-fluoro-phenyl)-2,5-dimethyl-2-trifluoromethyl-5,6-dihydro-2H-[1,4]oxazin-3-ylamine (1.035 g, 3.57 mmol) in H₂SO₄ (6 ml) was added in portions KNO₃ (0.379 g, 3.74 mmol) under ice-water cooling. The reaction mixture was stirred for 2 h at 25 °C, diluted with water and basified with K₂CO₃ under cooling. The product was extracted with EtOAc. Combined extracts were washed with saturated NaHCO₃ solution and brine, dried over MgSO₄, filtered and concentrated. Purification via chromatography on silica gel (hexane-EtOAc 4:1 to 1:1 containing 0.05% NEt₃) gave the title compound as a light yellow oil: TLC (hexane-EtOAc 1:1): Rf =0.50; HPLC Rt_{H5}= 0.749 min; ESIMS: 336 [(M+H)⁺]; ¹H NMR (360 MHz, CDCl₃): δ 8.48 (dd, 1 H), 8.14 (m, 1 H), 7.15 (dd, 1 H), 4.20 (br s, 2H), 4.04 (dd, 1 H), 3.91 (dd, 1 H), 1.54 (s, 3H), 1.49 (s, 3H).

### o) [(2R,5R)-5-(2-Fluoro-5-nitro-phenyl)-2,5-dimethyl-2-trifluoromethyl-5,6-dihydro-2H-[1,4]oxazin-3-yl]-carbamic acid tert-butyl ester

To a solution of (2R,5R)-5-(2-fluoro-5-nitro-phenyl)-2,5-dimethyl-2-trifluoromethyl-5,6-di-hydro-2H-[1,4]oxazin-3-ylamine (1.14 g, 3.4 mmol) in ACN (20 ml) was added Boc₂O (0.891 g, 4.08 mmol) and NEt₃ (0.72 ml, 5.1 mmol) and the mixture was stirred for 16 h at 25 °C. The reaction mixture was evaporated and the residual oil purified by chromatography on silica gel (hexane-EtOAc 20:1 to 7:3) to give the title compound after crystallization from Et₂O-hexane as beige crystals: TLC (hexane-EtOAc 3:1): Rf =0.37; HPLC Rt_{H5}= 1.355 min; ESIMS: 436 [(M+H)⁺]; ¹H NMR (360 MHz, CDCl₃): δ 11.04 (br s, 1 H), 8.24 (m, 2H), 7.30 (dd, 1 H), 4.41 (dd, 1 H), 4.11 (dd, 1 H), 1.68 (s, 3H), 1.51 (s, 9H), 1.49 (s, 3H).

### p) [(2R,5R)-5-(5-Amino-2-fluoro-phenyl)-2,5-dimethyl-2-trifluoromethyl-5,6-dihydro-2H-[1,4]oxazin-3-yl]-carbamic acid tert-butyl ester

A solution of [(2R,5R)-5-(2-fluoro-5-nitro-phenyl)-2,5-dimethyl-2-trifluoromethyl-5,6-dihydro-2H-[1,4]oxazin-3-yl]-carbamic acid tert-butyl ester (0.98 g, 2.25 mmol) in isopropanol-THF 2:1 (24 ml) was hydrogenated over 5% Pd-C for 4 h at 50 °C. The catalyst was filtered off over Celite and the filtrate was concentrated to provide the title compound after crystallization from TBME-hexane as beige crystals: TLC (hexane-EtOAc 1:1): Rf =0.42; HPLC Rt_{H5}= 0.955 min; ESIMS: 406 [(M+H)⁺]; ¹H NMR (360 MHz, CDCl₃): δ 6.82 (dd, 1 H), 6.52 (m, 2H), 4.30 (dd, 1H), 3.97 (dd, 1 H), 3.06 (br s, 2H), 1.58 (s, 3H), 1.48 (s, 3H), 1.46 (s, 9H).

### q) ((2R,5R)-5-{5-[(5-Bromo-pyrimidine-2-carbonyl)-amino]-2-fluoro-phenyl}-2,5-dimethyl-2-trifluoromethyl-5,6-dihydro-2H-[1,4]oxazin-3-yl)-carbamic acid tert-butyl ester

To a solution of [(2R,5R)-5-(5-amino-2-fluoro-phenyl)-2,5-dimethyl-2-trifluoromethyl-5,6-dihydro-2H-[1,4]oxazin-3-yl]-carbamic acid tert-butyl ester (76 mg, 0.187 mmol) in DMF (2 ml) was added 5-bromopyridine-2-carboxylic acid (47 mg, 0.225 mmol), EDC.HCl (48 mg, 0.244 mmol), HOAt (29 mg, 0.206 mmol) and DIPEA (0.08 ml, 0.469 mmol) and the reaction mixture was kept at 25 °C for 16 h. The mixture was concentrated, the residue dissolved in EtOAc and washed with saturated NaHCO₃ solution and brine, dried over MgSO₄, filtered and purified by chromatography on silica gel (hexane-EtOAc 20:1 to 1:1) to provide the title compound as a light yellow foam: HPLC Rt_{H5}= 1.297 min); ESIMS: 590, 592 [(M+H)⁺]; ¹H NMR (360 MHz, CDCl₃): δ 10.98 (br s, 1H), 9.71 (br s, 1H), 8.94 (s, 2H), 7.89 (m, 1 H), 7.49 (dd, 1 H), 7.12 (dd, 1H), 4.38 (d, 1 H), 4.04 (d, 1 H), 1.66 (s, 3H), 1.56 (s, 3H), 1.52 (s, 9H).

### r) 5-Bromo-pyrimidine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride

A solution of ((2R,5R)-5-{5-[(5-bromo-pyrimidine-2-carbonyl)-amino]-2-fluoro-phenyl}-2,5-dimethyl-2-trifluoromethyl-5,6-dihydro-2H-[1,4]oxazin-3-yl)-carbamic acid tert-butyl ester (90 mg, 0.153 mmol) in 4N HCl in dioxane (1 ml) was stirred at 40-45 °C for 6 h. The mixture was concentrated and the residue crystallized from Et₂O to yield the title compound as a beige solid: HPLC Rt_{H5}= 0.837 min); ESIMS: 490,492 [(M+H)⁺]; ¹H NMR (600 MHz, DMSO-d₆): 11.61 (br s, 1H), 11.14 (br s, 1 H), 9.61 (br s, 2H), 9,26 (s, 2H), 7.98 (d, 1 H), 7.90 (d, 1 H), 7.32 (dd, 1 H), 4.31 (d, 1 H), 4.10 (d, 1H), 1.72 (s, 3H), 1.62 (s, 3H).

### Example 72: can be prepared by procedures analogous to those used in reference example 71.

**Table 9**

| **Example** | **Compound** | **¹H-NMR (δ; DMSO-d₆)** | **MS [m/z; (M+1)⁺]** |
|---|---|---|---|
| **72** | 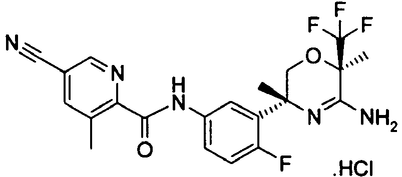 | 11.0 (s, 1H), 9.60 (d, 2H), 9.04 (s, 1H), 8.41 (s, 1H), 7.96 (m, 1H), 7.83 (dd, 1H), 7.33 (dd, 1H), 4.37 (d, 1H), 4.11 (d, 1H), 2.56 (s, 3H), 1.73 (s, 3H), 1.70 (s, 3H) | 450 |
| | 5-Cyano-3-methyl-pyridine-2-carboxylic acid [3-((3R,6R)-5-amino-3,6-dimethyl-6-trifluoro-methyl-3,6-dihydro-2H-[1,4]oxazin-3-yl)-4-fluoro-phenyl]-amide hydrochloride | | |

### More detailed description of preparation of Example 72: 5-Cyano-3-methyl-Pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride

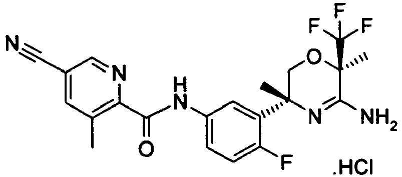

### a) ((2R,5R)-5-{5-[(5-Cyano-3-methyl-pyridine-2-carbonyl)-amino]-2-fluoro-phenyl}-2,5-dimethyl-2-trifluoromethyl-5,6-dihydro-2H-[1,4]oxazin-3-yl)-carbamic acid tert-butyl ester

To a solution of [(2R,5R)-5-(5-amino-2-fluoro-phenyl)-2,5-dimethyl-2-trifluoromethyl-5,6-dihydro-2H-[1,4]oxazin-3-yl]-carbamic acid tert-butyl ester (82 mg, 0.20 mmol) in DMF (2 ml) was added 5-cyano-3-methyl-pyridine-2-carboxylic acid [Acid-3] (42 mg, 0.26 mmol), EDC.HCl (51 mg, 0.26 mmol), HOAt (31 mg, 0.22 mmol) and DIPEA (0.09 ml, 0.52 mmol) and the reaction mixture was kept at 25 °C for 16 h. The mixture was concentrated, the residue dissolved in EtOAc and washed with saturated NaHCO₃ solution and brine, dried over MgSO₄, filtered and purified by chromatography on silica gel (hexane-EtOAc 20:1 to 1: 1) to provide the title compound as a light yellow foam: TLC (hexane-EtOAc 1:1): Rf =0.81; HPLC Rt_{H5}= 1.437 min; ESIMS: 550 [(M+H)⁺]; ¹H NMR (360 MHz, CDCl₃): δ 10.96 (br s, 1 H), 9.95 (br s, 1H), 8.63 (s, 2H), 7.88 (m, 1H), 7.71 (m, 1H), 7.54 (dd, 1H), 7.08 (dd, 1H), 4.34 (d, 1 H), 4.02 (d, 1 H), 2.77 (s, 3H), 1.63 (s, 3H), 1.47 (m, 12H).

### b) 5-Cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride

To a solution of ((2R,5R)-5-{5-[(5-cyano-3-methyl-pyridine-2-carbonyl)-amino]-2-fluoro-phenyl}-2,5-dimethyl-2-trifluoromethyl-5,6-dihydro-2H-[1,4]oxazin-3-yl)-carbamic acid tert-butyl ester in CH₂Cl₂ (0.3 ml) was added TFA (0.6 ml) and the reaction mixture was kept at 25 °C for 2 h. The reaction was added to cold 10% aqueous K₂CO₃ solution and the product extracted with EtOAc. Combined organic layers were washed with brine, dried over MgSO₄, filtered and concentrated to provide 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide as a colorless foam. The title compound was converted into its hydrochloride salt by dissolving the free base in CH₂Cl₂, adding 1 eq of 2N HCl in Et₂O, evaporation to dryness, followed by crystallization from CH₂Cl₂-Et₂O to provide the title compound as a white solid: HPLC Rt_{H5}= 0.957 min; ESIMS: 450 [(M+H)⁺]; ¹H NMR (600 MHz, DMSO-d₆): 11.0 (s, 1H), 9.60 (d, 2H), 9.04 (s, 1 H), 8.41 (s, 1 H), 7.96 (m, 1 H), 7.83 (dd, 1 H), 7.33 (dd, 1 H), 4.37 (d, 1H), 4.11 (d, 1 H), 2.56 (s, 3H), 1.73 (s, 3H), 1.70 (s, 3H).

### Example 72a: Preparation of crystalline 6-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide

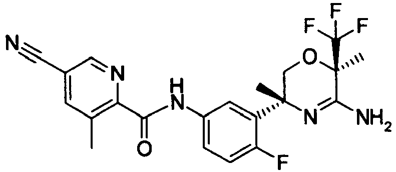

### a) 2-(5-Bromo-2-fluoro-phenyl)-propan-2-ol

To a solution of diisopropyl amine (57.3 ml, 402 mmol) in THF (500 ml) was added under argon a 1.6 M solution of nBuLi in hexane (260 ml, 416 mmol) below -50 °C. After stirring for 30 min at -75 °C, 4-bromo-1-fluoro benzene (31.1 ml, 277 mmol) was added while keeping the temperature below -70 °C. After stirring for 2 h at -75 °C, acetone (41.2 ml, 554 mmol) was added below -65 °C and the reaction mixture was stirred for 1 h at -75 °C, warmed up to -50 °C and poured onto 10% aqueous NH₄Cl solution. The mixture was extracted with TBME, organic phases were washed with aqueous KHSO₄ solution, saturated NaHCO₃ solution and brine, dried over MgSO₄, filtered and concentrated. The crude product was crystallized from hexane to provide the title compound as white crystals: TLC (hexane-EtOAc 3:1): Rf =0.45; UPLC Rt_{H5}=1.045 min; ¹H NMR (360 MHz, CDCl₃): δ 7.74 (dd, 1H), 7.36 (m, 1 H), 6.93 (dd, 1 H), 2.04 (d, 1 H), 1.63 (s, 6H).

### b) 4-Bromo-1-fluoro-2-isopropenyl-benzene

To a solution of 2-(5-bromo-2-fluoro-phenyl)-propan-2-ol (119.7g, 498 mmol) in CH₂Cl₂ (50 ml) was added hydrochinone (2.74 g, 24.9 mmol) and 250 ml 85% H₃PO₄. The resulting reaction mixture was stirred for 3.5 h at 50 °C. The mixture was poured onto ice-water and extracted with CH₂Cl₂. The organic phases were washed with 2N aqueous NaOH and water, dried over MgSO₄, filtered and concentrated. The crude product was dissolved in hexane and filtered through a plough of silica gel to obtain after concentration at 600 mbar the title compound as a colorless oil: TLC (hexane): Rf =0.52; UPLC Rt_{H5}=1.416 min; ¹H NMR (360 MHz, CDCl₃): δ 7.43 (dd, 1H), 7.37 (m, 1H), 6.94 (dd, 1H), 5.27 (d, 2H), 2.13 (s, 3H).

### c) (S)-2-(5-Bromo-2-fluoro-phenyl)-propane-1,2-diol

To a suspension of K₃Fe(CN)₆ (186 g, 561 mmol), K₂CO₃ (78 g, 561 mmol), (DHQ)₂-PHAL (1.311 g, 1.674 mmol) and K₂OₛO₂(OH)₄ (0.378 g, 1 mmol) in t-BuOH-H₂O 1:1 (1600 ml) was added 4-bromo-1-fluoro-2-isopropenyl-benzene (36 g, 167 mmol) at 0 °C and the reaction mixture was stirred for 14 h at 0 °C. After careful addition of Na₂S₂O₅ (100 g) at 0-5 °C the reaction mixture was stirred for 1 h before extraction with EtOAc. Combined extracts were washed with 5% NaS₃O₃ solution and brine, dried over MgSO4, filtered and concentrated to give the title compound as a white solid: TLC (hexane-EtOAc 1:1): Rf =0.46; UPLC Rt_{H5}=0.767 min; ESIMS: 266, 268 [(M+NH₄)⁺]; ¹H NMR (360 MHz, CDCl₃): δ 7.71 (dd, 1H), 7.27 (m, 1 H), 6.83 (dd, 1 H), 3.85 (d, 1H), 3.62 (d, 1H), 2.94 (s, 3H), 2.01 (s, 1 H), 1.43 (s, 3H).

### d) (S)-2-(5-Bromo-2-fluoro-phenyl)-2-methyl-oxirane

To a solution of (S)-2-(5-bromo-2-fluoro-phenyl)-propane-1,2-diol (37.35 g, 150 mmol) in CH₂Cl₂ (400 ml) was added under argon NEt₃ (41.8 ml, 300 mmol) and dropwise mesyl chloride (12.8 ml, 165 mmol) at 0-5 °C. After stirring for 0.5 h at 0-5 °C the reaction mixture was added to cold 1 N HCl and extracted with CH₂Cl₂. Combined extracts were washed with 1 N HCl, H₂O and saturated NaHCO₃ solution, dried over MgSO₄, filtered and concentrated. The crude mesylate was dissolved in TBME (500 ml) and 200 ml 2N aqueous NaOH and after stirring for 2 h at 25 °C the mixture was extracted with TBME. Combined extracts were washed with NaH₂PO₄ solution and brine, dried over MgSO₄, filtered and concentrated to provide the (S)-enantiomer as a colorless oil: 78% ee (Chiralpak AS-H 1218, hexane-EtOH 97:3, 0.4 mL/min); TLC (hexane-EtOAc 3:1): Rf =0.69; UPLC Rt_{H5}= 1.186 min; ¹H NMR (360 MHz, CDCl₃): δ 7.46 (dd, 1 H), 7.30 (m, 1 H), 6.83 (dd, 1H), 2.88 (d, 1 H), 2.72 (d, 1 H), 1.59 (s, 3H).

### e) (S)-1-Azido-2-(5-bromo-2-fluoro-phenyl)-propan-2-ol

To a solution of (S)-2-(5-bromo-2-fluoro-phenyl)-2-methyl-oxirane (51.85 g, 224 mmol) in EtOH (800 ml) was added NaN₃ (36.8 g, 531 mmol), NH₄Cl (60.6 g, 1122 mmol) and 18-crown-6 (59.8 g, 224 mmol) and the reaction mixture was heated at reflux for 6 h. The reaction mixture was filtered and concentrated to half of its volume. The residual oil was extracted with EtOAc. Combined extracts were washed with saturated NaHCO₃ solution and brine, dried over MgSO₄, filtered and concentrated to provide the title compound as a light yellow oil: TLC (hexane-EtOAc 1:1): Rf =0.70; UPLC Rt_{H3}= 1.115 min; ¹H NMR (360 MHz, CDCl₃): δ 7.72 (dd, 1 H), 7.32 (m, 1 H), 6.85 (dd, 1H), 3.73 (d, 1H), 3.51 (d, 1H), 2.44 (s, 1H), 1.50 (s, 3H).

### f) (S)-1-Amino-2-(5-bromo-2-fluoro-phenyl)-propan-2-ol

To a suspension of LiAlH₄ (4.65 g, 122 mmol) in THF (250 ml) was added under argon at 0-5 °C a solution of (S)-1-azido-2-(5-bromo-2-fluoro-phenyl)-propan-2-ol (33.4 g, 122 mmol) dissolved in THF (150 ml) over a period of 30 min. After stirring for 1 h at 0-5 °C, the reaction was quenched by careful addition of water (4.7 ml), 4 N NaOH (4.7 ml) and water (14.1 ml). After stirring for 3 h at 25 °C the white suspension was dried with MgSO₄, filtered and concentrated. The solidified product was re-crystallized from TBME-hexane to provide the title compound as beige crystals: 98% ee (Chiralpak AD-H hexane-EtOH 75-25 + 0.05% NEt₃); TLC (CH₂Cl₂-MeOH 10:1) Rf =0.10; UPLC Rt_{H5}= 0.558 min; ESIMS: 248, 250 [(M+H)⁺]; ¹H NMR (360 MHz, CDCl₃): δ 7.76 (dd, 1H), 7.25 (m, 1H), 6.82 (dd, 1H), 4.16 (br s, 1 H), 3.19 (d, 1H), 2.72 (d, 1H), 1.44 (s, 3H), 0.95 (br s, 2H).

### g) N-[(S)-2-(5-Bromo-2-fluoro-phenyl)-2-hydroxy-propyl]-2-nitro-benzenesulfonamide

To a solution of (S)-1-amino-2-(5-bromo-2-fluoro-phenyl)-propan-2-ol (34.7 g, 140 mmol) in THF (400 ml) was added 2-nitro-benzenesulfonyl chloride (34.9 g, 154 mmol) at 0-5 °C and afterwards 1 N aqueous NaOH over a period of 0.5 h. The reaction mixture was stirred for 2 h at 20 °C. The reaction mixture was diluted with TBME and washed with water and NaH₂PO₄ solution and brine, dried over MgSO₄, filtered and concentrated to provide the title compound after crystallization from TBME-hexane as beige crystals: TLC (toluene-EtOAc 3:1): Rf =0.51; UPLC Rt_{H5}= 1.118 min; ESIMS: 450, 452 [(M+NH₄)⁺]; ¹H NMR (360 MHz, CDCl₃): δ 7.98 (m, 1H), 7.81 (m, 1H), 7.65 (m, 2H), 7.59 (dd, 1H), 7.24 (m, 1 H), 6.79 (dd, 1 H), 5.60 (t, 1H), 4.16 (br s, 1 H), 3.55 (dd, 1H), 3.44 (dd, 1 H), 2.51 (s, 1 H), 1.51 (s, 3H).

### h) (R)-2-(5-Bromo-2-fluoro-phenyl)-2-methyl-1-(2-nitro-benzenesulfonyl)-aziridine

To a solution of N-[(S)-2-(5-bromo-2-fluoro-phenyl)-2-hydroxy-propyl]-2-nitro-benzenesulfonamide (20.8 g, 48 mmol) in CH₂Cl₂ (400 ml) was added PPh₃ (19.2 g, 72.4 mmol) at 0-5 °C and diethyl azodicarboxylate (11.6 ml, 72.4 mmol). The reaction mixture was stirred for 24 h at 25 °C and concentrated. The title compound was obtained after chromatographic purification over silica gel (hexane-EtOAc 20:1 to 2: 1) as yellow crystals: TLC (toluene-EtOAc 3:1): Rf =0.69; UPLC Rt_{H5}= 1.308 min; ¹H NMR (360 MHz, CDCl₃): δ 8.31 (m, 1H), 7.28 (m, 3H), 7.60 (dd, 1H), 7.42 (m, 1H), 6.91 (dd, 1H), 3.24 (s, 1H), 2.81 (s, 1 H), 2.06 (s, 3H).

### i) (R)-2-[(R)-2-(5-Bromo-2-fluoro-phenyl)-2-(2-nitro-benzenesulfonylamino)-propoxy]-3,3,3-trifluoro-2-methyl-propionic acid ethyl ester

To a suspension of NaH (2.53 g 60% in mineral oil, 63 mmol) in DMF (160 ml) was added drop-wise under argon (R)-3,3,3-trifluoro-2-hydroxy-2-methyl-propionic acid ethyl ester (11.99 g, 63 mmol) and after stirring for 0.5 h at 20 °C (R)-2-(5-bromo-2-fluoro-phenyl)-2-methyl-1-(2-nitro-benzenesulfonyl)-aziridine (21.85 g, 52.6 mmol). The reaction was kept at 25 °C for 16 h. The mixture was added to cold aqueous 2N HCl and the product extracted with TBME. Combined organic layers were washed with saturated NaHCO₃ solution and brine, dried over MgSO₄, filtered and concentrated. The residual solid was re-crystallized from TBME-hexane to provide the title compound as yellow crystals: TLC (hexane-EtOAc 1:1): Rf =0.59; UPLC Rt_{H5}= 1.444 min; ESIMS: 618, 620 [(M+NH₄)⁺]; ¹H NMR (360 MHz, CDCl₃): δ 7.83 (dd, 1H), 7.61 (m, 3H), 7.48 (dd, 1 H), 7.27 (m, 1 H), 6.73 (s, 1 H), 6.60 (dd, 1 H), 4.33 (m, 2H), 3.84 (s, 2H), 1.84 (s, 3H), 1.57 (s, 3H), 1.33 (t, 3H).

### j) (R)-2-[(R)-2-(5-Bromo-2-fluoro-pheyl)-2-(2-nitro-benzenesulfonylamino)-propoxy]-3,3,3-trifluoro-2-methyl-propionamide

A solution of (R)-2-[(R)-2-(5-bromo-2-fluoro-phenyl)-2-(2-nitro-benzenesulfonylamino)-propoxy]-3,3,3-trifluoro-2-methyl-propionic acid ethyl ester (26.6 g, 44.2 mmol) in 7N NH₃ in MeOH (75 ml) was stirred for 16 h at 50 °C. The solvent was removed under reduced pressure and the residual solid re-crystallized from Et₂O to give the title compound as yellow crystals: TLC (hexane-EtOAc 1:1): Rf =0.35; UPLC Rt_{H5}= 1.184 min; ESIMS: 589, 591 [(M+NH₄)⁺]; ¹H NMR (360 MHz, CDCl₃): δ 7.85 (d, 1 H), 7.64 (m, 3H), 7.44 (d, 1H), 7.41 (dd, 1 H), 7.26 (m, 1 H), 6.68 (br s, 1 H), 6.57 (dd, 1 H), 6.19 (s, 1 H), 5.54 (br s, 1 H), 4.24 (d, 1 H), 3.93 (d, 1 H), 1.79 (s, 3H), 1.67 (s, 3H).

### k) N-[(R)-1-(5-Bromo-2-fluoro-phenyl)-2-((R)-1-cyano-2,2,2-trifluoro-1-methyl-ethoxy)-1-methyl-ethyl]-2-nitro-benzenesulfonamide

To a solution of (R)-2-[(R)-2-(5-bromo-2-fluoro-phenyl)-2-(2-nitro-benzenesulfonylamino)-propoxy]-3,3,3-trifluoro-2-methyl-propionamide (20.83 g, 35.6 mmol) in CH₂Cl₂ (300 ml) was added under argon NEt₃ (12.5 ml, 89 mmol) and at 0-5 °C trifluoroacetic anhydride (6.15 ml, 42.7 mmol). After stirring for 4 h at 25 °C the reaction mixture was added to a cold NaHCO₃ solution and the product was extracted with CH₂Cl₂. Combined extracts were washed with cold 0.1 N aqueous HCl, water and saturated NaHCO₃ solution, dried over MgSO₄, filtered and concentrated to provide the title compound as a yellow oil, which was used as such for the next step: TLC (hexane-EtOAc 1:1): Rf =0.73; UPLC Rt_{H5}= 1.364 min; ESIMS: 571, 573 [(M+NH₄)⁺]; ¹H NMR (360 MHz, CDCl₃): δ 7.89 (d, 1 H), 7.62 (ddd, 1 H), 7.57 (ddd, 1H), 7.52 (m, 2H), 7.29 (m, 1H), 6.58 (dd, 1H), 6.19 (s, 1H), 4.17 (s, 2H), 1.81 (s, 3H), 1.72 (s, 3H).

### l) (2R,5R)-5-(5-Bromo-2-fluoro-phenyl)-2,5-dimethyl-2-trifluoromethyl-5,6-dihydro-2H-[1,4]oxazin-3-ylamine

To a solution of N-[(R)-1-(5-bromo-2-fluoro-phenyl)-2-((R)-1-cyano-2,2,2-trifluoro-1-methyl-ethoxy)-1-methyl-ethyl]-2-nitro-benzenesulfonamide (6.54 g, 11.8 mmol) and N-acetyl-cysteine (2.4 g, 26.0 mmol) in MeOH (80 ml) was added K₂CO₃ (3.62 g, 26.0 mmol) and the reaction mixture was heated at 80 °C for 16 h. After removal of the solvent the residue was dissolved in water and extracted with EtOAc. Combined extracts were washed with saturated NaHCO₃ solution and brine, dried over MgSO₄, filtered and concentrated to provide the title compound after chromatographic purification over silica gel (hexane-EtOAc 10:1 to 1:2 containing 0.03% NEt₃) as a yellow oil: TLC (hexane-EtOAc 1:1): Rf =0.58; UPLC Rt_{H5}= 0.843 min; ESIMS: 369, 371 [(M+H)⁺]; ¹H NMR (360 MHz, CDCl₃): δ 7.66 (dd, 1 H), 7.35 (m, 1 H), 6.91 (dd, 1H), 3.97 (m, 2H), 1.53 (s, 3H), 1.49 (s, 3H).

### m) (2R,5R)-5-(2-Fluoro-phenyl)-2,5-dimethyl-2-trifluoromethyl-5,6-dihydro-2H-[1,4]oxazin-3-ylamine

A solution of (2R,5R)-5-(5-bromo-2-fluoro-phenyl)-2,5-dimethyl-2-trifluoromethyl-5,6-dihydro-2H-[1,4]oxazin-3-ylamine (1.66 g, 4.5 mmol) and sodium acetate (0.369 g,4.5 mmol) in MeOH (50 ml) was hydrogenated over 10% Pd-C for 6 h at 50 °C. The catalyst was filtered off over Celite and the filtrate was concentrated. The residue was dissolved in saturated NaHCO₃ solution and extracted with EtOAc. Combined extracts were washed with brine, dried over MgSO₄, filtered and concentrated to provide the title compound as a colorless oil: TLC (hexane-EtOAc 1:1): Rf =0.19; UPLC Rt_{H5}= 0.777 min; ESIMS: 291 [(M+H)⁺]; ¹H NMR (360 MHz, CDCl₃): δ 7.41 (dt, 1 H), 7.26 (m, 1 H), 7.11 (t, 1 H), 7.05 (dd, 1 H), 4.11 (dd, 1 H), 3.94 (dd, 1 H), 1.54 (s, 3H), 1.49 (s, 3H).

### n) (2R,5R)-5-(2-Fluoro-5-nitro-phenyl)-2,5-dimethyl-2-trifluoromethyl-5,6-dihydro-2H-[1,4]oxazin-3-ylamine

To a solution of (2R,5R)-5-(2-fluoro-phenyl)-2,5-dimethyl-2-trifluoromethyl-5,6-dihydro-2H-[1,4]oxazin-3-ylamine (1.035 g, 3.57 mmol) in H₂SO₄ (6 ml) was added in portions KNO₃ (0.379 g, 3.74 mmol) under ice-water cooling. The reaction mixture was stirred for 2 h at 25 °C, diluted with water and basified with K₂CO₃ under cooling. The product was extracted with EtOAc. Combined extracts were washed with saturated NaHCO₃ solution and brine, dried over MgSO₄, filtered and concentrated. Purification via chromatography on silica gel (hexane-EtOAc 4:1 to 1:1 containing 0.05% NEt₃) gave the title compound as a light yellow oil: TLC (hexane-EtOAc 1:1): Rf =0.50; UPLC Rt_{H5}= 0.749 min; ESIMS: 336 [(M+H)⁺]; ¹H NMR (360 MHz, CDCl₃): δ 8.48 (dd, 1 H), 8.14 (m, 1 H), 7.15 (dd, 1H), 4.20 (br s, 2H), 4.04 (dd, 1H), 3.91 (dd, 1 H), 1.54 (s, 3H), 1.49 (s, 3H).

### o) [(2R,5R)-5-(2-Fluoro-5-nitro-phenyl)-2,5-dimethyl-2-trifluoromethyl-5,6-dihydro-2H-[1,4]oxazin-3-yl]-carbamic acid tert-butyl ester

To a solution of (2R,5R)-5-(2-fluoro-5-nitro-phenyl)-2,5-dimethyl-2-trifluoromethyl-5,6-dihydro-2H-[1,4]oxazin-3-ylamine (1.14 g, 3.4 mmol) in ACN (20 ml) was added Boc₂O (0.891 g, 4.08 mmol) and NEt₃ (0.72 ml, 5.1 mmol) and the mixture was stirred for 16 h at 25 °C. The reaction mixture was evaporated and the residual oil purified by chromatography on silica gel (hexane-EtOAc 20:1 to 7:3) to give the title compound after crystallization from Et₂O-hexane as beige crystals: TLC (hexane-EtOAc 3:1): Rf =0.37; UPLC Rt_{H5}= 1.355 min; ESIMS: 436 [(M+H)⁺]; ¹H NMR (360 MHz, CDCl₃): δ 11.04 (br s, 1H), 8.24 (m, 2H), 7.30 (dd, 1H), 4.41 (dd, 1H), 4.11 (dd, 1H), 1.68 (s, 3H), 1.51 (s, 9H), 1.49 (s, 3H).

### p) [(2R,5R)-5-(5-Amino-2-fluoro-phenyl)-2,5-dimethyl-2-trifluoromethyl-5,6-dihydro-2H-[1,4]oxazin-3-yl]-carbamic acid tert-butyl ester

A solution of [(2R,5R)-5-(2-fluoro-5-nitro-phenyl)-2,5-dimethyl-2-trifluoromethyl-5,6-dihydro-2H-[1,4]oxazin-3-yl]-carbamic acid tert-butyl ester (0.98 g, 2.25 mmol) in isopropanol-THF 2:1 (24 ml) was hydrogenated over 5% Pd-C for 4 h at 50 °C. The catalyst was filtered off over Celite and the filtrate was concentrated to provide the title compound after crystallization from TBME-hexane as beige crystals: TLC (hexane-EtOAc 1:1): Rf =0.42; UPLC Rt_{H5}= 0.955 min; ESIMS: 406 [(M+H)⁺]; ¹H NMR (360 MHz, CDCl₃): δ 6.82 (dd, 1H), 6.52 (m, 2H), 4.30 (dd, 1H), 3.97 (dd, 1H), 3.06 (br s, 2H), 1.58 (s, 3H), 1.48 (s, 3H), 1.46 (s, 9H).

### q) ((2R,5R)-5-{5-[(5-Cyano-3-methyl-pyridine-2-carbonyl)-amino]-2-fluoro-phenyl}-2,5-dimethyl-2-trifluoromethyl-5,6-dihydro-2H-[1,4]oxazin-3-yl)-carbamic acid tert-butyl ester

To a solution of [(2R,5R)-5-(5-amino-2-fluoro-phenyl)-2,5-dimethyl-2-trifluoromethyl-5,6-dihydro-2H-[1,4]oxazin-3-yl]-carbamic acid tert-butyl ester (2.2 g, 5.43 mmol) in DMF (20 ml) was added at 0-5 °C 5-cyano-3-methyl-pyridine-2-carboxylic acid (0.968 g, 5.97 mmol), EDC (1.095 g, 7.05 mmol), HOAt (1.182 g, 8.68 mmol) and the reaction mixture was stirred at 25 °C for 16 h. The reaction mixture was added to cold saturated NaHCO₃ solution and the product was extracted with TBME. Combined TBME layers were washed with H₂O and brine, dried over MgSO₄, filtered and concentrated to obtain after crystallization from diisopropylether the title compound as colorless crystals: UPLC Rt_{H5}=1.472 min); ESIMS: 550; [(M+H)⁺]; ¹H NMR (400 MHz, CDCl₃): δ 11.11 (s, 1H), 10.11 (s, 1 H), 8.79 (br s, 1 H), 8.01 (s, 1H), 7.84 (m, 1H), 7.68 (dd, 1 H), 7.21 (dd, 1H), 4.49 (d, 1 H), 4.16 (d, 1 H), 1.76 (s, 3H), 1.64 (s, 3H), 1.62 (s, 9H).

### r) 5-Cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide

To a solution of ((2R,5R)-5-{5-[(5-cyano-3-methyl-pyridine-2-carbonyl)-amino]-2-fluoro-phenyl}-2,5-dimethyl-2-trifluoromethyl-5,6-dihydro-2H-[1,4]oxazin-3-yl)-carbamic acid tert-butyl ester (2.27 g, 4.13 mmol) in CH₂Cl₂ (25 ml) was added TFA (41.3 mmol, 3.18 ml) and the reaction mixture was stirred at 25 °C for 2.5 h. The reaction mixture was added to 10% aqueous NaHCO₃ solution (pH >8) and the free base was extracted with EtOAc. Combined organic layers were washed with water and brine, dried over MgSO₄, filtered and concentrated. The crude product was re-crystallized twice by dissolving the material in EtOH (15 ml) at 50 - 60 °C and after saturation with H₂O (6-8 ml) the clear solution was allowed to cool to ambient temperature overnight to provide after filtration and drying the title compound in >99% purity as white crystals: UPLC Rt_{H5}= 0.905 min; ESIMS: 450 [(M+H)⁺]; ¹H NMR (600 MHz, DMSO-d₆): 10.72 (s, 1 H), 8.97 (s, 1 H), 8.39 (s, 1 H), 7.78 (d, 1 H), 7.71 (m, 1H), 7.15 (t, 1H), 6.08 (br s, 2H), 3.91 (d, 1H), 3.78 (d, 1 H), 2.52 (s, 3H), 1.46 (s, 3H), 1.41 (s, 3H).

### Example 72b: XRPD and DSC analysis of crystalline 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide

Crystalline 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide was analysed by XRPD and the ten most characteristic peaks are shown in Table 9.5 (see also Figure 2).

**Table 9.5**

| **Degrees 2-Θ** | **d-spacing (Å)** | **Intensity (counts)** | **Relative Intensity %** |
|---|---|---|---|
| 8.335 | 10.59904 | 22.60 | 7.1 Low |
| 9.032 | 9.78356 | 2013 | 6.3 Low |
| 10.851 | 8.14682 | 6590 | 20.7 Medium |
| 12.919 | 6.84703 | 7660 | 24.1 Medium |
| 13.859 | 6.38459 | 31816 | 100 High |
| 15.403 | 5.74799 | 15281 | 48.0 Medium |
| 16.211 | 5.46313 | 6577 | 20.7 Medium |
| 17.129 | 5.17238 | 8543 | 26.9 Medium |
| 18.158 | 4.88155 | 5135 | 16.1 Medium |
| 24.514 | 3.62838 | 10501 | 33 Medium |

X-ray powder diffraction (XRPD) analysis was performed using a Brucker D8 Advance x-ray diffractometer. Measurements were taken at about 30 kV and 40 mA under the following conditions:

| | |
|---|---|
| Scan rate (continuous scan): | 0.3 s/step (equals 107.1 s step time) |
| Step size: | 0.017° (2Theta) |
| Soller slit | 2.5° |
| Slits (from left to right): | V12 (variable), 6 mm antiscatter slit |

The X-ray diffraction pattern was recorded between 2° and 40° (2 theta) with CuK_{α} radiation for identification of the whole pattern.

Crystalline 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide was also analysed by differential scanning calorimetry (DSC) using a Q1000 DSC from TA instruments and found to have an onset of melting at about 94 °C (93.6 °C).

**Examples 112 and 144:** The compounds listed in Table 17 were prepared by procedures analogous to those used in Examples 42 or 112.

For enantiomerically pure compounds the racemic precursor [5-(5-amino-2-fluoro-phenyl)-5-difluoromethyl-5,6-dihydro-2H-[1,4]oxazin-3-yl]-carbamic acid tert-butyl ester (example 42j)) was separated via prep-HPLC on Chiralpak AD-H 250 x 4.6mm column using supercritical CO2 / EtOH 9 : 1 as an eluent. The desired compound was the slower eluting (R)-enantiomer. Enantiomeric excess = 99.7 %; [α]_{D} = -109.7° (c=1, CHCl₃).

**Table 17**

| **Example** | **Compound** | **¹H-NMR (δ; DMSO-d₆)** | **MS [m/z; (M+1)⁺]** |
|---|---|---|---|
| **112** | 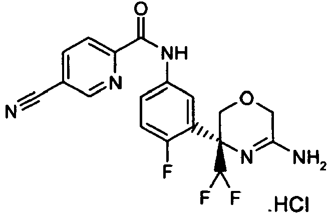 | 11.09 (s, 1H), 11.01 (s, 1H) 9.76 (s, 1H), 9.22 (s, 1H), 8.75 (s, 1H), 8.61 (d, 1H), 8.30 (d, 1H), 8.12 - 8.06 (m, 2H), 7.41 (dd, 1H), 6.79 (t, J = 54 Hz, 1H), 4.71 (d, 1H), 4.65 (d, 1H), 4.34 (d, 1H), 4.18 (d, 1H) | 390 |
| | 5-Cyano-pyridine-2-carboxylic acid [3-((R)-5-amino-3-difluoromethyl-3,6-dihydro-2H-[1,4]oxazin-3-yl)-4-fluoro-phenyl]-amide hydrochloride | | |
| **144** | 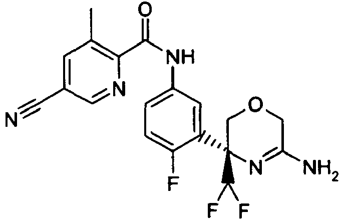 | 10.72 (s, 1H), 8.97 (s, 1H) 8.39 (s, 1H), 8.02 - 7.97 (m, 1H), 7.83 - 7.78 (m, 1H), 7.18 (dd, 1H), 6.14 (s, 2H), 6.14 (t, J = 54 Hz, 1H), 4.10 (d, 1H), 4.01 (d, 1H), 3.91 (d, 1H), 3.84 (d, 1H), 2.54 (s, 3H) | 404 |
| | 5-Cyano-3-methyl-pyridine-2-carboxylic acid [3-((R)-5-amino-3-difluoromethyl-3,6-dihydro-2H-[1,4]oxazin-3-yl)-4-fluoro-phenyl]-amide | | |

### More detailed description of preparation of Example 112: 5-Cyano-pyridine-2-carboxylic acid [3-((R)-5-amino-3-difluoromethyl-3,6-dihydro-2H-[1,4]oxazin-3-yl)-4-fluoro-phenyl]-amide hydrochloride

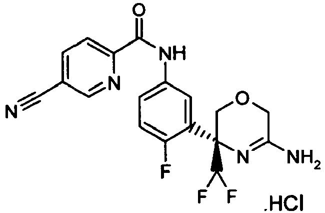

### a) 5-Difluoromethyl-5-(2-fluoro-phenyl)-morpholin-3-one

5-(5-Bromo-2-fluoro-phenyl)-5-difluoromethyl-morpholin-3-one (190 g, 586 mmol) [example 42 step e)] and sodium acetate (57.7 g, 703 mmol) were suspended in 1850 mL methanol. 10 % Pd on charcoal (18.7 g) was then added and the reaction mixture was shaken in a Parr apparatus in an atmosphere of hydrogen at rt. After 60 minutes the reaction mixture was filtered over celite and evaporated. The residue was dissolved in 2L TBME and washed with aqueous NaHCO₃ and brine. The organic layer was dried over MgSO₄.H₂O and evaporated to give 143.2 g of the title compound as a white solid.
HPLC: Rt_{H1} = 0.792 min; ESIMS [M+H]⁺= 246;
¹H-NMR (CDCl₃, 360 MHz): 7.50-7.43 (m, 2H), 7.32-7.27 (m, 1 H), 7.19 (dd, 1H), 6.62 (br, 1 H), 6.37 (t, J = 54 Hz, 1H), 4.34 (d, 1 H), 4.31 (d, 1H), 4.22 (d, 1H), 4.20 (d, 1H).

### b) 5-Difluoromethyl-5-(2-fluoro-phenyl)-morpholine-3-thione

A mixture of 5-difluoromethyl-5-(2-fluoro-phenyl)-morpholin-3-one (141 g, 575 mmol) and Lawesson's reagent (132 g, 316 mmol) in 1400 ml of THF was heated at 68°C for 1 h, cooled down and then evaporated. The residue was dissolved in 1 L DCM and filtered over 2 Kg silica gel with 10 L DCM to give 161 g of the title compound in the form of a greenish resin that slowly crystallized. The compound was used without further purification.
HPLC: Rt_{H1} = 1.799 min; ESIMS [M+H]⁺= 262; ¹H-NMR (360 MHz, CDCl₃): 7.42-7.35 (m, 1H), 7.28 (t, 1 H), 7.19 (t, 1 H), 7.11 (dd, 1 H), 6.29 (t, J = 54 Hz, 1 H), 4.57 (d, 1 H), 4.47 (d, 1H), 4.21 (d, 1 H), 4.18 (d,1H).

### c) 5-Difluoromethyl-5-(2-fluoro-phenyl)-5,6-dihydro-2H-[1,4]oxazin-3-ylamine

5-Difluoromethyl-5-(2-fluoro-phenyl)-morpholine-3-thione (160 g, 570 mmol) was dissolved in 2.4 L of a NH₃ solution 7 mol/L in methanol for 6.5 h and afterwards left standing overnight. The reaction mixture was evaporated and taken up in 2 L 1 N aqueous HCl and 2 L TBME. The aqueous phase was washed with TBME and made basic by the addition of 300 ml 30% aqueous NaOH and some ice. The mixture was extracted with DCM three times and the combined organic layers were dried with Na₂SO₄ and concentrated in vacuo. The title compound was obtained by crystallization from DCM/ heptanes (128.45 g).
HPLC: Rt_{H3}= 2.059 min; ESIMS [M+H]⁺= 245;
¹H-NMR (CDCl₃, 360 MHz): 7.77 (t, 1 H), 7.38 - 7.30 (m, 1 H), 7.21 (t, 1 H), 7.09 (dd, 1 H), 6.19 (t, J = 54 Hz, 1H), 4.51 (br, 2H), 4.32, (d, 1H), 4.18 (d, 1 H), 4.05 (d, 1 H), 3.96 (d, 1H), 1.39 (s, 3H), 1.24 (s, 3H).

### d) 5-Difluoromethyl-5-(2-fluoro-5-nitro-phenyl)-5,6-dihydro-2H-[1,4]oxazin-3-ylamine

Potassium nitrate (60.3 g, 596 mmol) was added portionwise to 600 ml sulfuric acid (Temperature < 20°C). This solution was added dropwise to a solution of 5-difluoromethyl-5-(2-fluoro-phenyl)-5,6-dihydro-2H-[1,4]oxazin-3-ylamine (112 g, 459 mmol) in 600 ml sulfuric acid, while keeping the reaction temperature < 22°C with an ice bath. After stirring for 1 h, the mixture was poured onto 10 Kg ice. TBME (6 L) was added and the pH was adjusted to 12-14 by the addtion of about 5 L 30% aqueous NaOH. The phases were separated and the aqueous phase was extracted twice with TBME. The compined organic layers were dried with sodium sulfate and evaporated to give 130 g of a yellow solid that was used further without purification.
HPLC: Rt_{H3} = 2.063 min; ESIMS [M+H]⁺ = 290;
¹H-NMR (CDCl₃, 360 MHz): 8.71 (dd, 1 H), 8.13 (dt, 1 H), 7.13 (dd, 1 H), 5.99 (t, J = 54 Hz, 1H), 4.55 (br, 2H), 4.33 (dd, 1 H), 4.10 (d, 1 H), 3.97 (d, 1 H), 3.82 (dt, 1 H).

### e) [5-Difluoromethyl-5-(2-fluoro-5-nitro-phenyl)-5,6-dihydro-2H-[1,4]oxazin-3-yl]-carbamic acid tert-butyl ester

A solution of 5-Difluoromethyl-5-(2-fluoro-5-nitro-phenyl)-5,6-dihydro-2H-[1,4]oxazin-3-ylamine (144.5 g, 500 mmol), Boc anhydride (142 g, 650 mmol) and DIPEA (131 ml, 749 mmol) in 2500 ml THF was stirred for 3 days at rt, after which there was still starting material remaining. Boc anhydride (56 g, 325 mmol) was added, the mixture heated to 60°C and stirred for 10h until the reaction was complete. The mixture was evaporated, dissolved in TBME, washed with ice-cold 1 N aqueous HCl, water, 10% aqueous NaHCO₃ and brine. The organic phase was dried with sodium sulfate, filtered and evaporated. The product was purified by crystallization from DCM/ heptanes. Yield 182.8 g white crystalls.
HPLC: Rt_{H1} = 3.259 min; ESIMS [M+Na]⁺= 412;
¹H-NMR (CDCl₃, 360 MHz): 8.70 (dd, 1 H), 8.27 (dt, 1 H), 7.34 (br, 1 H), 7.25 (dd, 1 H), 6.09 (t, J = 54 Hz, 1H), 4.85 (d, 1 H), 4.58 (d, 1 H), 4.49 (dd, 1 H), 3.94 (dt, 1 H).

### f) [5-(5-Amino-2-fluoro-phenyl)-5-difluoromethyl-5,6-dihydro-2H-[1,4]oxazin-3-yl]-carbamic acid tert-butyl ester

[5-Difluoromethyl-5-(2-fluoro-5-nitro-phenyl)-5,6-dihydro-2H-[1,4]oxazin-3-yl]-carbamic acid tert-butyl ester (180 g, 462 mmol) and 17.61 g Pd-C 10% were suspended in 1760 mL THF. The mixture was shaken in a Parr apparatus in an atmosphere of hydrogen at rt. After 6 h the reaction mixture was filtered over celite and evaporated. The residue was crystallized from DCM/heptanes to provide 157.6 g of the title compound as beige crystals.
HPLC: Rt_{H3} = 2.748 min; ESIMS [M+H]⁺ = 360;
¹H-NMR (CDCl₃, 360 MHz): Spectrum uninterpretable due to the presence of a complex mixture of rotamers.

### g) [(R)-5-(5-Amino-2-fluoro-phenyl)-5-difluoromethyl-5,6-dihydro-2H-[1,4]oxazin-3-yl]-carbamic acid tert-butyl ester

The racemic product ((rac)[5-(5-Amino-2-fluoro-phenyl)-5-difluoromethyl-5,6-dihydro-2H-[1,4]oxazin-3-yl]-carbamic acid tert-butyl ester) was separated via prep-HPLC on Chiralpak AD-H 20um (8 x 100 x 48mm HPLC colums), on a Bayer SMB CC50 instrument using SMB technology with heptane/EtOH/MeOH 70 : 20 : 10 as eluent. The desired compound was the slower eluting (R)-enantiomer. Yielding 72.29 g of the title compound as a colourless foam. ee = 99.3 %; Opt. rotation: [α]_{D} -97.5° (c=1, CHCl₃)
HPLC: Rt_{H3}= 2.748 min; ESIMS [M+H]⁺= 360;
¹H-NMR (CDCl₃, 360 MHz): Spectrum uninterpretable due to the presence of a complex mixture of rotamers.

### h) ((R)-5-{5-[(5-Cyano-pyridine-2-carbonyl)-amino]-2-fluoro-phenyl}-5-difluoromethyl-5,6-dihydro-2H-[1,4]oxazin-3-yl)-carbamic acid tert-butyl ester

[(R)-5-(5-Amino-2-fluoro-phenyl)-5-difluoromethyl-5,6-dihydro-2H-[1,4]oxazin-3-yl]-carbamic acid tert-butyl ester (35 g, 97.4 mmol), 5-cyano-pyridine-2-carboxylic acid (15.87 g, 107.14 mmol) and HOBt hydrate (22.35 g, 146.1 mmol) were dissolved in 185 ml DMF and stirred with ice cooling. When the temperature had reached 0-5°C EDC (22.33 ml, 126.62 mmol) was added dropwise. The mixture was stirred for 2 h. The ice bath was taken away and stirring was continued for 2h. The mixture was taken up in EtOAc and water. The phases were separated and the organic phase was washed with 5% aqueous NaHCO₃ and brine. The organic phase was dried with MgSO₄.H₂O and evaporated to provide a beige solid. Crystallisation from EtOAc/hexane gave the title compound as colorless crystals. Yield 44.47 g.
HPLC: Rt_{H1}= 2.888 min; ESIMS [M+Na]⁺= 512;
¹H-NMR (CDCl₃, 360 MHz, signals broadened due to rotamers): 8.95 (s, 1 H), 8.48 (d, 1H), 8.25 (d, 1H), 8.08-8.03 (m, 1H), 7.84-7.80 (m, 1H), 7.37 (s, 1H), 7.17 (t, 1 H), 6.18 (t, J = 54 Hz, 1 H), 4.83 (d, 1 H), 4.60 (d, 1 H), 4.42 (d, 1 H), 4.4-4.3 (br, 1 H), 3.97 (d, 1H), 1.53 (s, 9H).

### i) 5-Cyano-pyridine-2-carboxylic acid [3-((R)-5-amino-3-difluoromethyl-3,6-dihydro-2H-[1,4]oxazin-3-yl)-4-fluoro-phenyl]-amide

((R)-5-{5-[(5-Cyano-pyridine-2-carbonyl)-amino]-2-fluoro-phenyl}-5-difluoromethyl-5,6-dihydro-2H-[1,4]oxazin-3-yl)-carbamic acid tert-butyl ester (44.47 g, 91.0 mmol) was dissolved in 450 ml DCM and mildly chilled with a rt water bath. TFA (150 ml) was added. The reaction was slightly exothermic. The mixture was stirred for 1.5 h at rt. The volatiles were removed with vacuum at rt. The residue was taken up in DCM and the procedure repeated twice. The residue was taken up in 3 L EtOAc and washed with 10% aqueous Na₂CO₃ and brine. The organic phase was dried with sodium sulfate and partially evaporated. iPrOH was added and the mixture chilled. The title compound was collected as snow-white crystals. Yield 30.56 g.
HPLC: Rt_{H3}= 2.605 min; ESIMS [M+H]⁺= 390;
¹H-NMR (dmso-d6, 600 MHz): 10.85 (s, 1H), 9.22 (s, 1H), 8.58 (d, 1 H), 8.27 (d, 1H), 8.18-8.14 (m, 1 H), 7.85-7.80 (m, 1H), 7.19 (t, 1 H), 6.16 (br s, 2H), 6.14 (t, J = 54 Hz, 1 H), 4.12 (d, 1 H), 4.01 (d, 1 H), 3.92 (d, 1H), 3.88 (d, 1 H).

### j) 5-Cyano-pyridine-2-carboxylic acid [3-((R)-5-amino-3-difluoro-methyl-3,6-dihydro-2H-[1,4]oxazin-3-yl)-4-fluoro-phenyl]-amide, hydrochloride

A solution of 5-cyano-pyridine-2-carboxylic acid [3-((R)-5-amino-3-difluoro-methyl-3,6-dihydro-2H-[1,4]oxazin-3-yl)-4-fluoro-phenyl]-amide (277 mg, 0.71 mmol) in 5 ml THF was triturated with 0.9 ml of 1 M HCl in Et₂O. The mixture was partially evaporated, diluted with TBME and partially evaporated (3x), finally to dryness. The hydrochloride salt contained a significant amount of THF. It was taken up in EtOH and evaporated to dryness twice. The product was finally lyophilized with 15 ml water. Yield 261 mg white lyophilisate.
¹H-NMR (dmso-d6, 600 MHz): 11.05 (s, 1 H), 11.01 (s, 1 H), 9.75 (s, 1 H), 9.25 (s, 1 H), 8.73 (br s, 1 H), 8.61 (d, 1 H), 8.10 (d, 1 H), 8.12-8.07 (m, 2H), 7.41 (dd, 1 H), 6.79 (t, J = 54 Hz, 1 H), 4.70 (d, 1 H), 4.65 (d, 1 H), 4.36 (d, 1 H), 4.18 (d, 1H).

### First alternative procedure for the preparation of Example 144: 5-Cyano-3-methyl-pyridine-2-carboxylic acid [3-((R)-5-amino-3-difluoromethyl-3,6-dihydro-2H-[1,4]oxazin-3-yl)-4-fluoro-phenyl]-amide

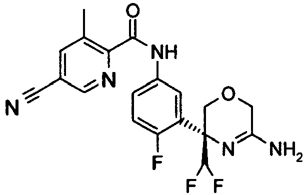

### a) 1-(5-Bromo-2-fluoro-phenyl)-2,2-difluoro-ethanone

To a solution of diisoprpopylamine (343 g, 3.39 mol) in THF (4 L) was added slowly n-BuLi (1.4 L, 3.5 mol, 2.5 M in hexane) at -78°C while keeping reaction temperature under -55°C. After addition, the mixture was stirred at -78 °C for 15 min. At this point, a solution of 4-bromo-1-fluorobenzene (540 g, 3.08 mol) in THF (1 L) was cooled to -60 °C before adding to the reaction mixture gradually while keeping reaction temperature under -55 °C and the resulting yellow solution was stirred at -78 °C for 150 min. Ethyldifluoroacetate (421 g, 3.39 mol) was added to the reaction mixture over 15 min and then the mixture was stirred at -78 °C for 15 min. 1 M HCl (6.0 L) was added to the reaction mixture with stirring. Aqueous layer was extracted with TBME (3 x 4 L). The combined organic phases were washed with water, dried (MgSO₄) and evaporated in vacuo. The resulting residue was distilled under vacuum (90 - 95 °C/0.19 mbar) and fraction at 54 ~ 62 °C was collected to obtain the title compound as a colorless liquid. ESIMS: 253 [(M+H)⁺]; ¹H NMR (400 MHz, CDCl₃): δ 8.06 (dd, 1 H), 7.75 (ddd, 1 H), 7.13 (dd, 1H), 6.41 (dt, 1 H).

### b) 2-Methyl-propane-2-sulfinic acid [1-(5-bromo-2-fluoro-phenyl)-2,2-difluoro-eth-(Z)-ylidene]-amide

To the mixture of 1-(5-bromo-2-fluoro-phenyl)-2,2-difluoro-ethanone (6.0 kg, 23.7 mol) and (S)-(-)-tert-butanesulfinamide (3.3 kg, 27.2 mol) were added toluene (73 L) with stirring at ambient temperature. After 5 min, titanium ethoxide (6.5 kg, 28.5 mol) was added and the mixture was heated to 50 - 55 °C. The mixture was stirred at 50 - 55 °C for 3 h and allowed to cool to ambient temperature before concentration *in vacuo.* The resulting crude product was used directly in next chemical transformation without further purification. ESIMS: 356 [(M+H)⁺].

### c) (Sₛ)-2-Methyl-propane-2-sulfinic acid [(S)-1-(5-bromo-2-fluoro-phenyl)-1-difluoromethyl-allyl]-amide

To a solution of vinyl magnesium bromide (400 mL, 400 mmol, 1 M in THF) was added toluene (400 mL) and the resulting mixture was cooled to below -65 °C. To the mixture was added a solution of crude 2-methyl-propane-2-sulfinic acid [1-(5-bromo-2-fluoro-phenyl)-2,2-difluoro-eth-(Z)-ylidene]-amide (40.2 g, 113 mmol) in toluene (200 mL) within 20 min, which was pre-cooled to below -60 °C, while keeping the reaction temperature under -65 °C. After addition, the resulting mixture was stirred at -65 - -72 °C for 40 min before quenching with aqueous H₂SO₄ solution (270 ml, 8.4 wt %). The resulting mixture was stirred and warmed slowly to -20°C, another portion of aqueous H₂SO₄ solution (540 ml, 8.4 wt %) was added to the mixture slowly via syringe, the resulting mixture was warmed and stirred at 10 ~ 20 °C for 1 hour. The two layers were separated and the organic layer was washed with aqueous sodium bicarbonate solution (540 mL, 4 wt %), followed by brine. The organic layer was filtered through a pad of Celite^{®}, concentrated in vacuo to obtain crude title compound as a 3:1 mixture of diastereoisomers, which was used in the following step without further purification. ESIMS: 384 [(M+H)]⁺.

### d) 2-Methylpropane-2-sulfinic acid [(R)-1-(5-bromo-2-fluorophenyl)-2,2-difluoro-1-hydroxymethylethyl]-amide

To the solution of (Sₛ)-2-methyl-propane-2-sulfinic acid [(S)-1-(5-bromo-2-fluoro-phenyl)-1-difluoromethyl-allyl]-amide (20 g, 52 mmol) in CH₂Cl₂ (640 mL) and MeOH (160 mL) was added potassium acetate (1 g, 10.4 mmol) at ambient temperature. The stirred mixture was then cooled to -70 °C. O₂ gas was bubbled through the reaction mixture with stirring via the gas dispenser under the surface of reaction mixture for 15 min at 1 L/min flow rate. 02 was then replaced by ozone and bubbled through with stirring at -70 °C till the reaction mixture turned blue. At this point, ozone was stopped and replaced by bubbling O₂ through the reaction mixture till the fade of blue color. Then sodium borohydride (3.9 g, 104 mmol) was added in portions while maintaining the reaction mixture at < -60 °C. The mixture was allowed to warm to -20 °C over 1 h with stirring. Quantofix^{®} Peroxide 25 test stick (Sigma Aldrich) was used to determine the leftover of peroxide in the reaction mixture. When no peroxide exists in the reaction mixture revealed by peroxide test stick, saturated aqueous NH₄Cl solution was added slowly to the reaction mixture with stirring till finish of H2 gas evolving. The mixture was concentrated in vacuo to remove organic volatiles. The aqueous residue was partitioned between ethyl acetate and H₂O. The separated organic layer was concentrated *in vacuo* to give the crude title compound as a 3:1 mixture of diastereoisomers. The oily crude title compound was dissolved in TBME and the solution was heated to 50~ 55 °C before addition of n-heptane at 50 - 55 °C.The mixture was allowed to cool gradually over 20 min to 40 - 45°C. At this point, pure title compound seed was added to the flask and the reaction mixture was cooled gradually with gentle stirring over 2 h to 0 - 5 °C and then stirred at 0 - 5 °C for another 8 h. The resulting slurry was filtered and the filter cake was washed with cold mixed solvent (TBME:n-heptane = 1:2). The filter cake was dried in vacuum oven at 40 °C for 12 h to give pure title compound as a white crystalline solid. ESIMS: 388 [(M+H)⁺]; ¹H NMR (400 MHz, DMSO-d₆): δ 7.80 (dd, 1H), 7.62 (m, 1H), 7.21 (dd, 1 H), 6.51 (t, 1 H), 5.72 (s, 1H), 5.23 (dd, 1 H), 3.97 (d, 1 H), 1.18 (s, 9H).

### e) (R)-2-Amino-2-(5-bromo-2-fluoro-phenyl)-3,3-difluoro-propan-1-ol

To a solution of 2-methyl-propane-2-sulfinic acid [(R)-1-(5-bromo-2-fluoro-phenyl)-2,2-difluoro-1-hydroxymethyl-ethyl]-amide (10g, 25.76 mmol) in DCM (100ml) was added under argon a 30% HCl in IPA solution (10 ml, 91.6 mmol) below 25 °C. After stirring for 30 min at 25 °C, cooled down to -10°C, filtered and dried to provide the title compound HCl salt as white crystals: ESIMS: 284, 286 [(M+H)⁺], 1H NMR (400 MHz, DMSO-d₆): δ 9.42 (b, 3H), 7.84 (m, 1 H), 7.74 (m, 1 H), 7.35 (dd, 1 H), 6.68 (t, 1H), 4.03 (m, 2H).

### f) (R)-5-Difluoromethyl-5-(2-fluoro-phenyl)-morpholin-3-one

To a suspension of (R)-2-amino-2-(5-bromo-2-fluoro-phenyl)-3,3-difluoro-propan-1-ol HCl salt (0.5 g, 1.56 mmol) in IPAc (5 ml) was added under argon a solution of K₂CO₃ (0.862g, 6.24 mmol) in 5ml water below 25 °C. After stirring for 30 min at 25 °C to provide a diphase clear solution, cooled down to -5°C, a solution of 2-chloroacetyl chloride(0.41g, 3.59mmol) in IPAc (2 mL) was added slowly with the temperature below 10°C. After stirring for 30 min at 20 °C, the organic phase was collected and used for next step directly. THF (3 ml) was added to the above solution, the mixture was cooled to 0°C. The solution was added under argon KOtBu (0.35g, 3.12mmol) below 10°C. After stirring at 20°C for 3h, water was added to quench the reaction. The organic phase was washed by 1 N HCl water solution (5ml). The organic phase was collected and used for next step directly. The above solution was added water (5ml) and Pd/C(10%, 50mg), the mixture was stirred under H2 (1.5 bar) at 25 °C for 3h. The catalyst was filtered out. The filtration was washed by 15% NaCl water solution. The solvent was removed under vacuum to provide the title compound as yellowish solid. ESIMS: 246 [(M+H)⁺]. 1 H NMR (400 MHz, DMSO-d₆): δ 9.08 (s, 1 H), 7.57 (m, 1 H), 7.48 (m, 1H), 7.30 (m, 1 H), 7.81 (m, 1 H), 7.30 (m, 2H), 6.55(t, 1 H), 4.1(m, 4H).

### g) (R)-5-Difluoromethyl-5-(2-fluoro-phenyl)-5,6-dihydro-2H-[1,4]oxazin,3-ylamine sulphate

To a solution of (R)-5-difluoromethyl-5-(2-fluoro-phenyl)-morpholin-3-one (120 g, 489.4 mmol) in CH₂Cl₂ (1.2 L) was added P₂S₅ (108 g, 489.4 mmol) at room temperature. The resulted yellow suspension was heated to reflux for 3.5 h. The reaction mixture was filtered through a pad of silica gel (200 - 300 mesh), washed with CH₂Cl₂ for 3 times to get a brown solution. The solvent was removed to provide the crude product as an orange solid, which was dispersed in THF (100 mL), then 25% NH₃ water solution (750 mL) was added at room temperature. 27 hours later, the reaction mixture was extracted with IPAC. The combined organic phase was washed with water, concentrated to provide a yellow oil. The crude product was dissolved in IPAC (1.2 L) and MeOH (60 mL) and some solid undissolved was removed through filtration. Then conc. H₂SO₄ (57.6 g, 587.3 mmol) was added to get a white suspension. The suspension was stirred at room temperature overnight and filtered to provide the title compound as a white solid: ESIMS: 245; [(M-H₂SO₄+H)⁺]; ¹H NMR (400 MHz, DMSO-d₆): δ 9.60 (bs, 1 H), 8.82 (br s, 1H), 7.52 (m, 2H), 7.35 (m, 2H), 6.75 (t, 1H), 4.65 (dd, 2H), 4.36 (d, 1 H), 4.14 (d, 1H).

### h) (R)-5-Difluoromethyl-5-(2-fluoro-5-nitro-phenyl)-5,6-dihydro-2H-[1,4]oxazin-3-ylamine

To a solution of (R)-5-difluoromethyl-5-(2-fluoro-phenyl)-5,6-dihydro-2H-[1,4]oxazin-3-ylamine sulphate (127.2 g, 371.6 mmol) in conc. H₂SO₄ (380 mL) was added at 5-15°C HNO₃ (25.8 g, 408.8 mmol). After addition, the reaction mixture was added slowly to 12% NH₃ water solution (2.6 L) at 10-20 °C. The resulted suspension (pH-8) was filtered to provide the title compound as a yellow solid: ESIMS: 290; [(M+H)⁺]; ¹H NMR (400 MHz, CDCl₃): δ 8.89 (m, 1H), 8.14 (m, 1H), 7.18 (dd, 1H), 6.10 (t, 1 H), 5.65 (br s, 2H), 4.57 (dd, 1H), 4.13 (dd, 2H), 3.88 (m, 1H).

### i) [(R)-5-Difluoromethyl-5-(2-fluoro-5-nitro-phenyl)-5,6-dihydro-2H-[1,4]oxazin-3-yl]-carbamic acid tert-butyl ester

To a suspension of (R)-5-difluoromethyl-5-(2-fluoro-5-nitro-phenyl)-5,6-dihydro-2H-[1,4]oxazin-3-ylamine (20 g, 64.659 mmol) in 2-Me-THF (20 mL) was added at room temperature DIPEA (9.831 g, 76.066 mmol) and (Boc)₂O (18.111 g, 82.984 mmol). The resulted mixture was heated to 60-65 °C and stirred at this temperature for 17 h. n-Heptane (200 mL) was added to the reaction mixture, then the mixture was cooled to -20 °C. The suspension was filtrated to provide the title compound as a yellow solid: ESIMS: 390; [(M+H)⁺]; ¹H NMR (400 MHz, CDCl₃): δ 8.67 (m, 1H), 8.23 (m, 1H), 7.23 (t, 1 H), 6.06 (t, 1H), 4.83 (d, 1 H), 4.55 (d, 1 H), 4.46 (dd, 1H), 3.91 (d, 1 H), 1.50 (s, 9H).

### j) [(R)-5-(5-Amino-2-fluoro-phenyl)-5-difluoromethyl-5,6-dihydro-2H-[1,4]oxazin-3-yl]-carbamic acid tert-butyl ester

To a suspension of [(R)-5-difluoromethyl-5-(2-fluoro-5-nitro-phenyl)-5,6-dihydro-2H-[1,4]oxazin-3-yl]-carbamic acid *tert*-butyl ester (8 g, 19.23 mmol) in MeOH (56 mL) was added Pd(OH)₂/C (20%, 50% water, 0.8 g). The atmosphere in the reactor was replaced by hydrogen (1 bar), then the reaction mixture was stirred at 20 °C for 5 h, filtered through Micro-crystals cellulose and concentrated. The crude product was recrystallized from MeOH and water to provide the title compound as a white solid: ESIMS: 360.1521; [(M+H)⁺]; ¹H NMR (400 MHz, DMSO-*d*₆): δ 9.99 (s, 1 H), 6.83 (dd, 1 H), 6.74 (m, 1H), 6.50 (m, 1 H), 6.22 (t, 1H), 4.95 (s, 2H), 4.46 (dd, 2H), 4.03 (m, 1 H), 3.84 (m, 1H), 1.42 (s, 9H).

### k) ((R)-5-{5-[(5-Cyano-3-methyl-pyridine-2-carbonyl)-amino]-2-fluoro-phenyl}-5-difluoromethyl-5,6-dihydro-2H-[1,4]oxazin-3-yl)-carbamic acid tert-butyl ester

To a mixture of [(R)-5-(5-amino-2-fluoro-phenyl)-5-difluoromethyl-5,6-dihydro-2H-[1,4]oxazin-3-yl]-carbamic acid tert-butyl ester (636 g, 1.77 mol) and 5-cyano-3-methylpyridine-2-carboxylic acid (301 g, 1.86 mol) was added THF (4.2 L) under N2 atmosphere at ambient temperature. To the stirring mixture was added N-methylmorpholine (428 mL, 3.89 mol) and the mixture was stirred to form a homogeneous solution. The reaction solution was cooled to 0-5°C before addition of isobutyl chloroformate (256 mL, 1.95 mol) via an addition funnel while keeping the reaction temperature under 10°C. After the addition, the mixture was stirred at 0-10°C for 1 h before filtration. The filtrate was evaporated in vacuo and the resulting residue was dissolved in the mixture of CH₂Cl₂ (3 L) and methanol (1 L). The mixture was concentrated in vacuo at 400 mbar/35 °C till no more volatile coming out to give a slurry. The slurry was filtered and the filter cake was washed with methanol till the wash getting colorless. The wet cake was dried in a vacuum oven at 45 °C for 24 h to give title compound as a white solid: ESIMS: 504; [(M+H)⁺]; ¹H NMR (400 MHz, DMSO-d₆): δ 10.72 (s, 1H), 10.00 (s, 1H), 8.98 (s, 1 H), 8.39 (s, 1 H), 7.92 (m, 1H), 7.81 (m, 1 H), 7.24 (m, 1H), 6.30 (t, 1 H), 4.56 (d, 1 H), 4.44 (d, 1H), 4.12 (m, 1 H), 3.90 (m, 1H), 2.55 (s, 3H), 1.41 (s, 9H).

### I) 5-Cyano-3-methyl-pyridine-2-carboxylic acid [3-((R)-5-amino-3-difluoromethyl-3,6-dihydro-2H-[1,4]oxazin-3-yl)-4-fluoro-phenyl]-amide

To a solution of ((R)-5-{5-[(5-cyano-3-methyl-pyridine-2-carbonyl)-amino]-2-fluoro-phenyl}-5-difluoromethyl-5,6-dihydro-2H-[1,4]oxazin-3-yl)-carbamic acid tert-butyl ester (23g, 45.77 mmol) in DCM (615 ml) was added under argon TFA (23 ml, 302.27 mmol) below 25 °C. After stirring for 18h at 25 °C, another TFA (12 ml, 151.13 mmol) was added. After stirring at 25 °C for another 6 h, cooled down to -5° C, quenched by 12.5% NH3 aqueous solution (110 ml). The DCM phase was collected and water (100 ml) was added. The DCM of the diphase solution was removed to provide a yellowish suspension. Filtered and dried to provide the title compound as yellowish crystals. ESIMS: 404 [(M+H)⁺]; 1 H NMR (400 MHz, DMSO- *d*₆): 10.72 (s, 1H), 8.96 (s, 1 H), 8.38 (s, 1 H), 7.99 (dd, 1 H), 7.81 (m, 1 H), 7.18 (d, 1 H), 6.17 (br s, 2H), 6.15 (t, 1 H), 3.98 (m, 4H), 2.53 (s, 3H).

The 5-cyano-3-methylpyridine-2-carboxylic acid used in step k was prepared as follows:

### m) 5-Bromo-3-methyl-pyridine-2-carboxylic acid tert-butyl ester

To the stirred pale yellow solution of 5-bromo-3-methylpyridine-2-carboxylic acid (20 g, 92.6 mmol), DMAP (0.57 g, 4.6 mmol) in DMF (120 mL) was added Boc anhydride (20.2 g, 92.6 mmol) at ambient temperature. The reaction mixture was stirred at 35 - 40 °C for 2 h before addition of another portion of Boc anhydride (20.2 g, 92.6 mmol). The reaction was stirred at 35 - 40 °C for another 16 h to achieve full conversion to the title compound, which was used directly in the following step without further purification.
ESIMS: 272 [(M+H)+].

### n) 5-Cyano-3-methylpyridine-2-carboxylic acid tert-butyl ester

To a solution of 5-bromo-3-methyl-pyridine-2-carboxylic acid tert-butyl ester (48 g, 176 mmol) in DMF (253 mL) under N₂ atmosphere was sequentially added Zn powder (1.15 g, 17.6 mmol), Pd₂(dba)₃ (4.04 g, 4.4 mmol), tri-tert-butylphosphine (214 g, 10.6 mmol, 10 wt% in n-hexane). The mixture was then heated to 50 - 60 °C with stirring, at this point, Zn(CN)₂ (14.5 g, 123.5 mmol) was added to the mixture in one portion. The reaction mixture was then heated at 69 °C with stirring. After 1 h, the reaction mixture was allowed to cool to below 30 °C and filtered. The filter cake was washed with DMF. To the filtrate was added 2-methyltetrahydrofuran with stirring and the mixture was cooled to < 20 °C, to the mixture was added slowly 3N aqueous ammonia solution with stirring and the mixture was then stirred vigorously for 30 min at 20 - 30 °C before layer separation. The organic layer was washed with brine and concentrated *in vacuo* to give the title compound as a yellowish crystalline solid: ESIMS: 219; [(M+H)⁺]; ¹H NMR (400 MHz, acetone-*d*₆): δ 8.79 (d, 1H), 8.20 (d, 1H), 2.49 (s, 3H), 1.60 (s, 9H).

### o) 5-Cyano-3-methylpyridine-2-carboxylic acid

To the stirred solution of 5-cyano-3-methylpyridine-2-carboxylic acid tert-butyl ester (20.7 g, 94.9 mmol) in toluene (200 mL) was added H₂O (3.4 mL) at ambient temperature. The mixture was then cooled to 0~5 °C with stirring, at this point, TFA (200 mL) was added to the mixture slowly while maintaining the reaction temperature at 25 - 35 °C. The reaction mixture was then heated at 33 °C with stirring. After 60 min, to the reaction mixture was added toluene and the mixture was concentrated *in vacuo* to ~1/4 of the original volume to remove TFA by azeotropic distillation with toluene. To the concentrated mixture was added toluene and the mixture was evaporated *in vacuo* to ~1/4 of the original volume. The same evaporation manipulation was repeated once and the precipitated product was collected by filtration. The filter cake was washed with toluene and dried in vacuum oven at 50 °C for 8 h to give the title compound as a pale yellow crystalline solid: ESIMS: 163; [(M+H)⁺]; ¹H NMR (400 MHz, DMSO-*d*₆): δ 8.90 (s, 1H), 8.35 (s, 1H), 2.46 (s, 3H).

### Second alternative procedure for the preparation of Example 144: 5-Cyano-3-methyl-Pyridine-2-carboxylic acid [3-((R)-5-amino-3-difluoromethyl-3,6-dihydro-2H-[1,4]oxazin-3-yl)-4-fluoro-phenyl]-amide

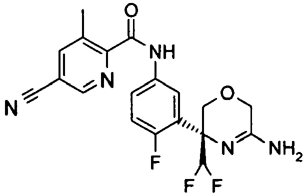

### a) (R)-5-(5-Bromo-2-fluoro-phenyl)-5-difluoromethyl-5,6-dihydro-2H-[1,4]oxazin-3-ylamine

To a solution of (R)-5-(5-bromo-2-fluoro-phenyl)-5-difluoromethyl-morpholin-3-one (10 g, 30.86 mmol) in CH₂Cl₂ (100 mL) was added P₂S₅ (6.86 g, 30.86 mmol) at room temperature. The resulted yellow suspension was heated to reflux for 6.5 h. The reaction mixture was filtered through a pad of silica gel (200 - 300 mesh), washed with CH₂Cl₂ for 3 times to get a brown solution. The solvent was removed to provide the crude product as a yellow solid, which was dispersed in THF (10 mL), then 25% NH₃ water solution (60 mL) was added at room temperature. 20 hours later, the reaction mixture was extracted with IPAC. The combined organic phase was washed with brine, dried over Na₂SO₄, filtered and concentrated to provide the title compound as a yellow solid.
ESIMS: [(M+H)+] 323; 1 H NMR (400 MHz, d6-DMSO): δ 7.98 (dd, 1 H), 7.54 (m, 1H), 7.17 (m, 1 H), 6.32 (br s, 2H), 6.10 (t, 1H), 4.24 (dd, 1H), 3.98 (dd, 2H), 3.73 (m, 1H).

### b) 5-Bromo-3-methyl-pyridine-2-carboxylic acid tert-butyl ester

To a stirred pale yellow solution of 5-bromo-3-methylpyridine-2-carboxylic acid (20 g, 92.6 mmol), DMAP (0.57 g, 4.6 mmol) in DMF (120 mL) was added Boc anhydride (20.2 g, 92.6 mmol) at ambient temperature. The reaction mixture was stirred at 35 - 40 °C for 2 h before addition of another portion of Boc anhydride (20.2 g, 92.6 mmol). The reaction was stirred at 35 - 40 °C for another 16 h to achieve full conversion to the title compound, which was used directly in the following step without further purification.
ESIMS: 272 [(M+H)+].

### c) 5-Cyano-3-methylpyridine-2-carboxylic acid tert-butyl ester

To a solution of 5-bromo-3-methyl-pyridine-2-carboxylic acid tert-butyl ester (48 g, 176 mmol) in DMF (253 mL) under N₂ atmosphere was sequentially added Zn powder (1.15 g, 17.6 mmol), Pd₂(dba)₃ (4.04 g, 4.4 mmol), tri-tert-butylphosphine (214 g, 10.6 mmol, 10 wt% in n-hexane). The mixture was then heated to 50 - 60 °C with stirring, at this point, Zn(CN)₂ (14.5 g, 123.5 mmol) was added to the mixture in one portion. The reaction mixture was then heated at 69 °C with stirring. After 1 h, the reaction mixture was allowed to cool to below 30 °C and filtered. The filter cake was washed with DMF. To the filtrate was added 2-methyltetrahydrofuran with stirring and the mixture was cooled to < 20 °C, to the mixture was added slowly 3N aqueous ammonia solution with stirring and the mixture was then stirred vigorously for 30 min at 20 - 30 °C before layer separation. The organic layer was washed with brine and concentrated *in vacuo* to give the title compound as a yellowish crystalline solid: ESIMS: 219; [(M+H)⁺]; ¹H NMR (400 MHz, acetone-d₆): δ 8.79 (d, 1H), 8.20 (d, 1H), 2.49 (s, 3H), 1.60 (s, 9H).

### d) 5-Cyano-3-methylpyridine-2-carboxylic acid

To the stirred solution of 5-cyano-3-methylpyridine-2-carboxylic acid tert-butyl ester (20.7 g, 94.9 mmol) in toluene (200 mL) was added H₂O (3.4 mL) at ambient temperature. The mixture was then cooled to 0-5 °C with stirring, at this point, TFA (200 mL) was added to the mixture slowly while maintaining the reaction temperature at 25 - 35 °C. The reaction mixture was then heated at 33 °C with stirring. After 60 min, to the reaction mixture was added toluene and the mixture was concentrated *in vacuo* to ~1/4 of the original volume to remove TFA by azeotropic distillation with toluene. To the concentrated mixture was added toluene and the mixture was evaporated *in vacuo* to ~1/4 of the original volume. The same evaporation manipulation was repeated once and the precipitated product was collected by filtration. The filter cake was washed with toluene and dried in vacuum oven at 50 °C for 8 h to give the title compound as a pale yellow crystalline solid: ESIMS: 163; [(M+H)⁺]; ¹H NMR (400 MHz, DMSO-*d*₆): δ 8.90 (s, 1H), 8.35 (s, 1 H), 2.46 (s, 3H).

### e) 5-Cyano-3-methyl-pyridine-2-carboxylic acid methyl ester

To a solution of 5-cyano-3-methylpyridine-2-carboxylic acid (2 g, 12.4 mmol) in methanol (20 mL) was added thionyl chloride dropwise under reflux. The mixture was then heated at reflux with stirring. After 60 min, HPLC analysis revealed completion of reaction. To the reaction mixture was added toluene and the mixture was concentrated *in vacuo* to give the title compound as a pale yellow solid: ESIMS: 177; [(M+H)⁺];¹H NMR (400 MHz, DMSO-*d*₆): δ 8.92 (s, 1H), 8.39 (s, 1H), 3.90 (s, 3H), 2.46 (s, 3H).

### f) 5-Cyano-3-methyl-pyridine-2-carboxylic acid amide

To a solution of 5-cyano-3-methylpyridine-2-carboxylic acid methyl ester (1 g, 5.6 mmol) in methanol (5 mL) was added ammonia (4 mL, 28 mmol, 7N solution in methanol). The mixture was then heated at 50 °C with stirring in a sealed tube. After 60 min, HPLC analysis revealed completion of reaction. The mixture was concentrated *in vacuo* to give the title compound as a pale yellow solid: ESIMS: 162; [(M+H)⁺]; ¹H NMR (400 MHz, DMSO-*d*₆): 8.87 (d, 1 H), 8.30 (d, 1 H), 8.09 (br s, 1 H), 7.74 (br s, 1 H), 2.50 (s, 3H)

### g) 5-Cyano-3-methyl-pyridine-2-carboxylic acid [3-((R)-5-amino-3-difluoromethyl-3,6-dihydro-2H-[1,4]oxazin-3-yl)-4-fluoro-phenyl]-amide

50 mg of (R)-5-(5-bromo-2-fluoro-phenyl)-5-difluoromethyl-5,6-dihydro-2H-[1,4]oxazin-3-ylamine, 30 mg of 5-cyano-3-methyl-pyridine-2-carboxylic acid amide, 3.0 mg Cul and 65 mg K₃PO₄ was charged to the reactor under N₂ atomosphere. 1.0 ml of dioxane was added. Followed by 3.0 mg of N1,N2-dimethylethane-1,2-diamine. The resulting reaction mixture was heated to reflux and hold for 23h. Cooled down to 23°C then dilute with 15 ml IPAc. The organic phase was washed with water (5 ml x 2), concentrated to dry under reduced pressure, Purification via column chromatography on silica gel (IPAc-Heptane 2:1 to 3:1) gave 30 mg of white solid. ESIMS: [M+H]+ = 403.9, ¹H NMR (400 MHz, DMSO-*d*₆): 10.72 (s, 1 H), 8.96 (s, 1 H), 8.38 (s, 1 H), 7.99 (dd, 1 H), 7.81 (m, 1 H), 7.18 (d, 1 H), 6.17 (br s, 2H), 6.15 (t, 1 H), 3.98 (m, 4H), 2.53 (s, 3H).

### Example 144a: preparation of 5-cyano-3-methyl-pyridine-2-carboxylic acid [3-((R)-5-amino-3-difluoromethyl-3,6-dihydro-2H-[1,4]oxazin-3-yl)-4-fluoro-phenyl]-amide hydrochloride hydrate

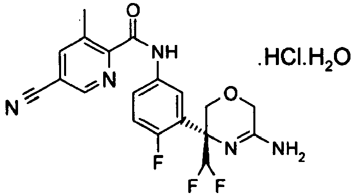

2.3g of 5-cyano-3-methyl-pyridine-2-carboxylic acid [3-((R)-5-amino-3-difluoromethyl-3,6-dihydro-2H-[1,4]oxazin-3-yl)-4-fluoro-phenyl]-amide (5.702mml) was put in a 4neck flask. In an Erlenmeyer flask 92ml acetonitrile and 4.6ml water were added. The mixture was shaken for a few seconds and 561.6mg of HCl 37% (5.702mmol) were added. The solution was shaken again for a few seconds and then it was added to the 4neck flask in portions. The mixture was stirred with a paddle and it was heated in a water bath up to 85°C. At 85°C all the solid material was completely dissolved and a slightly yellow clear solution was obtained. The solution was left at rt to cool down with stirring. Precipitate appeared at 40°C. The suspension was let to stir overnight and it was filtered after 16 hours through a Büchner filter. The isolated material was dried in an oven at rt for 16h and 1854.5mg of a white powder was obtained.

### Example 152: Crystalline 5-cyano-pyridine-2-carboxylic acid [3-((R)-5-amino-3-difluoromethyl-3,6-dihydro-2H-[1,4]oxazin-3-yl)-4-fluoro-phenyl]-amide

5-cyano-pyridine-2-carboxylic acid [3-((R)-5-amino-3-difluoromethyl-3,6-dihydro-2H-[1,4]oxazin-3-yl)-4-fluoro-phenyl]-amide was dissolved in EtOAc, isopropanol added and the resulting solution concentrated at reduced pressure. This procedure was repeated until most of the product had crystallised.
The resultant crystalline material was analysed by XRPD and the ten most characteristic peaks are shown in Table 18 (see also Figure 1).

**Table 18**

| **Degrees 2-Θ** | **d-spacing (Å)** | **Intensity (counts)** | **Relative Intensity %** |
|---|---|---|---|
| 8.29 | 10.65649 | 9840 | High |
| 10.813 | 8.17512 | 5198 | Medium |
| 14.077 | 6.28645 | 1911 | Low |
| 14.525 | 6.09337 | 2446 | Low |
| 16.624 | 5.32842 | 19854 | High |
| 18.919 | 4.68693 | 3766 | Medium |
| 21.453 | 4.13863 | 3862 | Medium |
| 22.244 | 3.99323 | 6947 | Medium |
| 23.327 | 3.81033 | 4257 | Medium |
| 25.436 | 3.49889 | 3672 | Medium |
| 28.495 | 3.12985 | 5558 | Medium |

X-ray powder diffraction (XRPD) analysis was performed using a Brucker D8 Advance x-ray diffractometer. Measurements were taken at about 30 kV and 40 mA under the following conditions:

| | |
|---|---|
| Scan rate (continuous scan): | 0.3 s/step (equals 107.1 s step time) |
| Step size: | 0.017° (2Theta) |
| Soller slit | 2.5° |
| Slits (from left to right): | V12 (variable), 6 mm antiscatter slit |

The X-ray diffraction pattern was recorded between 2° and 40° (2 theta) with CuK_{α} radiation for identification of the whole pattern.

The crystalline material was also analysed by differential scanning calorimetry using a PerkinElmer DSC7 and was found to have an onset of melting at about 227°C (227.46°C).

### Preparation of Intermediates

The substituted acid building blocks were either commercially available or can be prepared as described in the literature or in an analogous manner, e.g. WO 2005063738, WO 2009091016, WO 2010047372, Bioorg. Med. Chem. 2001, 9, 2061-2071, or can be prepared as described hereafter or in an analogous manner.

### Acid-1: 5-Cyano-4,6-dideutero-3-trideuteromethyl-pyridine-2-carboxylic acid

### a) 5-Bromo-4,6-dideutero-3-trideuteromethyl-pyridine-2-carboxylic acid

A suspension of 2.16 g (0.00 mmol) 5-bromo-3-methyl-pyridine-2-carboxylic acid in 36 ml of D2O (99,96% D) was treated with 4 ml of a 40% solution of NaOD in D2O. The homogeneous solution was heated in a 100 ml Teflon vessel with a Synthos 3000 Microwave apparatus. The mixture was heated at 160°C for 5 h and cooled down. 1 H-NMR and MS analises of the product showed that deuteration had progressed to a high degree. Only minor amounts of tetradeutero derivatives were present. The reaction mixture was acidified to pH3 with 2N HCl and extracted with EtOAc. The organic phase was dried with MgSO4.H2O and evaporated to give the title compound as a white solid, pure enough for further transformations.
HPLC: Rt_{H2}= 2.829 min; ESIMS [M+H]⁺= 221, 223 (1 Br, 5D);

### b) 5-Bromo-4,6-dideutero-3-trideuteromethyl-pyridine-2-carboxylic acid tert-butyl ester

A solution of 1.65 g (7.46 mmol) 5-bromo-4,6-dideutero-3-trideuteromethyl-pyridine-2-carboxylic acid and two drops of DMF were dissolved in 17 ml DCM. Oxalyl chloride (1.3 ml, 14.9 mmol) was added dropwise. The development of gas started immediately. After stirring for 2 h at 25°C the mixture was evaporated, taken up in toluene and evaporated again. The residual brownish resin was dissolved in 3ml THF and added to a stirred solution of 14 ml (22.39 mmol) BuLi (1.6 M in hexane) in 24 ml t-BuOH. After 1 h the mixture was poured onto 10% aqueous NH4Cl and extracted wth TBME. The organic layer was washed with brine, dried with MgSO4.H2O and evaporated. Chromatography on silica gel (hexane/EtOAc 9:1) provided the title compound as a colorless liquid.
HPLC: Rt_{H1}= 3.002 min; ESIMS [M+H]⁺ = 277, 279 (1 Br, 5D);
¹H-NMR (360 MHz, CDCl₃): 1.65 (s, 9H).

### c) 5-Cyano-4,6-dideutero-3-trideuteromethyl-pyridine-2-carboxylic acid tert-butyl ester

A mixture of 1.41 g (5.09 mmol) 5-bromo-4,6-dideutero-3-trideuteromethyl-pyridine-2-carboxylic acid tert-butyl ester, 0.418 g (3.56 mmol) Zn(CN)2, 0.033 g Zn powder (0.509 mmol) and 0.265 g (0.254 mmol) Pd2(dba)3.CHCl3 were suspended in 14 ml DMF under nitrogen atmosfere. A 0.25 M solution of tBu3P in dioxane (4.0 ml, 1.02 mmol) was added and the mixture was stirred for 16 h at 60°C. After being cooled down the mixture was diluted with TBME, filtered over celite and washed with brine three times. The crude product was purified by column chromatography on silica gel (hexane/EtOAc 5-15%) to give the title compound as an off white solid.
HPLC: Rt_{H3}= 3.275 min; ESIMS [M+Na]⁺ = 246 (5D);
¹H-NMR (360 MHz, CDCl₃): 1.68 (s, 9H);
Ft-IR: 2231 cm⁻¹ (CN).

### d) 5-Cyano-4,6-dideutero-3-trideuteromethyl-pyridine-2-carboxylic acid

To a solution of 825 mg (3.69 mmol) 5-cyano-4,6-dideutero-3-trideuteromethyl-pyridine-2-carboxylic acid tert-butyl ester in 5.1 g (37 mmol) 1,3-dimethoxybenzene were added 8.3 ml TFA and stirred for 6.5 h. The reaction mixture was diluted with toluene and evaporated. The residue was taken up in toluene and evaporated (2x). The product was crystallized from TBME/hexane to give the title compound as a white powder.
HPLC: Rt_{H2}= 2.397 min; ESIMS [M+H]⁺ = 168 (5D);
¹H-NMR (360 MHz, CDCl₃): non-deuterated impurities.

### Acid-3: 5-Cyano-3-methyl-pyridine-2-carboxylic acid

The title compound was prepared by an analogous procedure to Acid-1 starting with 5-bromo-3-methyl-pyridine-2-carboxylic acid instead of the deuterated derivative [Acid-1 step a)].
Rf (hexanes /EtOAc 6: 1) = 0.28
¹H-NMR (360 MHz, CDCl₃): 8.09 (dd, 1 H), 7.79 (ddd, 1 H), 7.17 (t, 1H), 6.44 (t, J = 45 Hz, 1 H).

### Example 186: Biological activity of compounds of the formula I

The compounds of the Examples hereinbefore show the following IC₅₀ values in Test 1 described hereinbefore:

**Table 22**

| Example | Bace IC50 [µM] |
|---|---|
| 72 | 0.010 |
| 112 | 0.042 |
| 144 | 0.017 |

Certain compounds of the Examples hereinbefore show the following IC₅₀ values in Test 4 described hereinbefore:

**Table 23**

| Example | Bace IC50 [µM] |
|---|---|
| 72 | 0.003 |
| 112 | 0.007 |
| 144 | 0.006 |

### Figure Descriptions

Figure 1 shows the X-ray powder diffraction pattern for a crystalline form of 5-cyano-pyridine-2-carboxylic acid [3-((R)-5-amino-3-difluoromethyl-3,6-dihydro-2H-[1,4]oxazin-3-yl)-4-fluoro-phenyl]-amide when measured using CulK_{α} radiation. For details see Example 152.
Figure 2 shows the X-ray powder diffraction pattern for crystalline 5-cyano-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide when measured using CuK_{α} radiation. For details see Example 72b.

## Claims

1. A compound selected from the group consisting of: 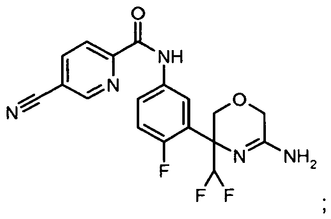 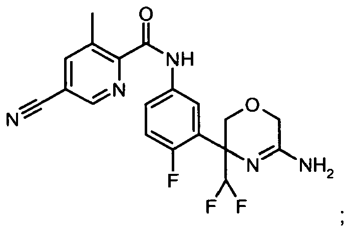 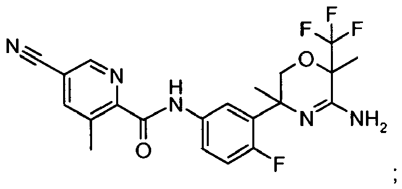 and pharmaceutically acceptable salts thereof.

2. A compound according to Claim 1, or a pharmaceutically acceptable salt thereof, having the following formula

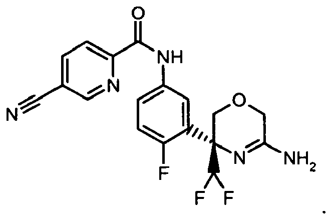

3. A compound according to Claim 1, or a pharmaceutically acceptable salt thereof, having the following formula

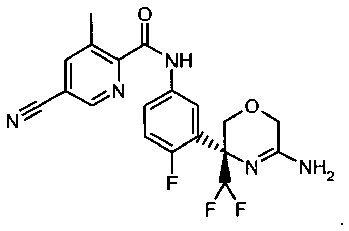

4. A compound according to Claim 1, or a pharmaceutically acceptable salt thereof, having the following formula

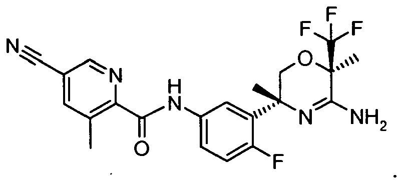

5. A compound according to any one of Claims 1 to 4 in free form.

6. A compound according to any one of Claims 1 to 4 in pharmaceutically acceptable salt form.

7. A compound according to any one of Claims 1 to 4 in hydrochloride salt form.

8. A pharmaceutical composition comprising a compound according to any one of Claims 1 to 7, or a pharmaceutically acceptable salt thereof, as active ingredient and a pharmaceutical carrier or diluent.

9. A compound according to any one of Claims 2 to 4, or a pharmaceutically acceptable salt thereof, for use as a medicament.

10. A compound according to any one of Claims 2 to 4, or a pharmaceutically acceptable salt thereof, for use in the treatment of Alzheimer's Disease.

## Patentansprüche

1. Verbindung, ausgewählt aus der Gruppe, bestehend aus: 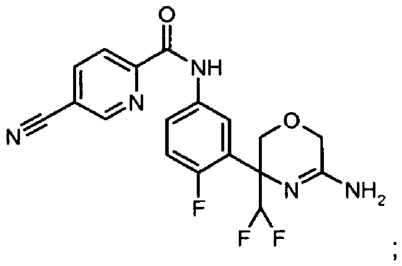 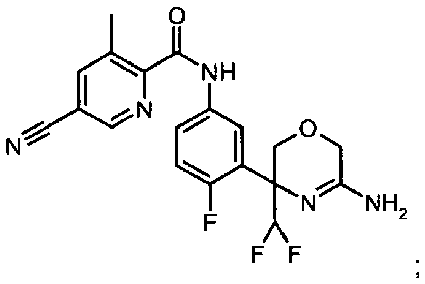 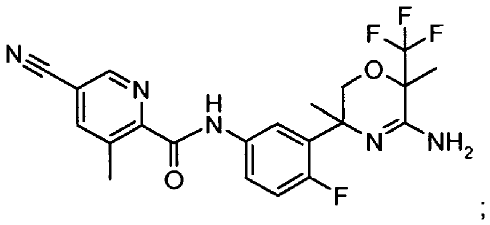 und pharmazeutisch akzeptable Salze davon.

2. Verbindung nach Anspruch 1 oder ein pharmazeutisch akzeptables Salz davon mit der folgenden Formel:

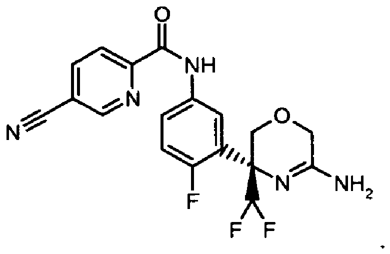

3. Verbindung nach Anspruch 1 oder ein pharmazeutisch akzeptables Salz davon mit der folgenden Formel:

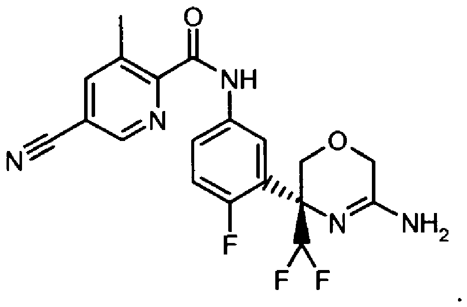

4. Verbindung nach Anspruch 1 oder ein pharmazeutisch akzeptables Salz davon mit der folgenden Formel:

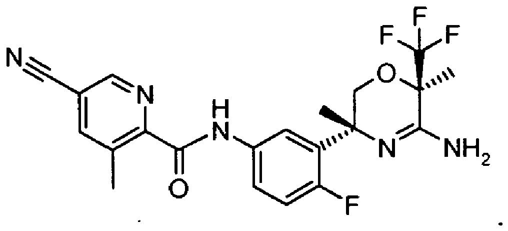

5. Verbindung nach einem der Ansprüche 1 bis 4 in freier Form.

6. Verbindung nach einem der Ansprüche 1 bis 4 in Form eines pharmazeutisch akzeptablen Salzes.

7. Verbindung nach einem der Ansprüche 1 bis 4 in Form eines Hydrochloridsalzes.

8. Pharmazeutische Zusammensetzung, umfassend eine Verbindung nach einem der Ansprüche 1 bis 7 oder ein pharmazeutisch akzeptables Salz davon als Wirkstoff und einen pharmazeutisch akzeptablen Träger oder ein pharmazeutisch akzeptables Verdünnungsmittel.

9. Verbindung nach einem der Ansprüche 2 bis 4 oder ein pharmazeutisch akzeptables Salz davon zur Verwendung als Medikament.

10. Verbindung nach einem der Ansprüche 2 bis 4 oder ein pharmazeutisch akzeptables Salz davon zur Verwendung bei der Behandlung von Morbus Alzheimer.

## Revendications

1. Composé choisi dans le groupe consistant en : 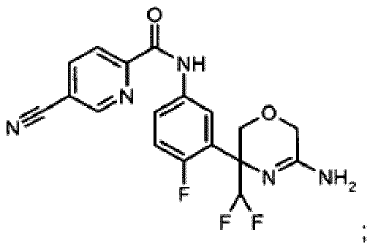 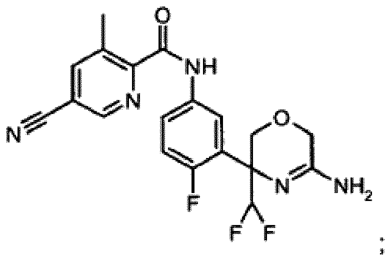 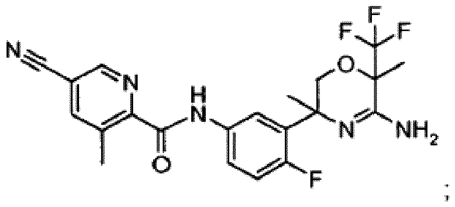 et sels pharmaceutiquement acceptables de ce composé.

2. Composé selon la revendication 1, ou sel pharmaceutiquement acceptable de celui-ci, ayant la formule suivante :

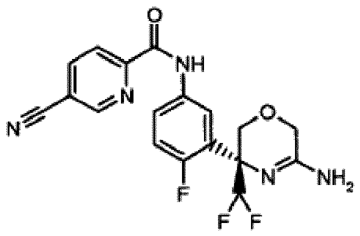

3. Composé selon la revendication 1, ou sel pharmaceutiquement acceptable de celui-ci, ayant la formule suivante :

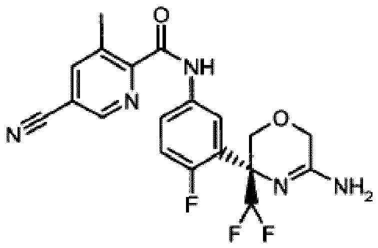

4. Composé selon la revendication 1, ou sel pharmaceutiquement acceptable de celui-ci, ayant la formule suivante :

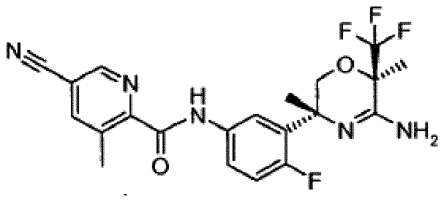

5. Composé selon l'une quelconque des revendications 1 à 4, sous forme libre.

6. Composé selon l'une quelconque des revendications 1 à 4, sous forme de sel pharmaceutiquement acceptable.

7. Composé selon l'une quelconque des revendications 1 à 4, sous forme de sel chlorhydrate.

8. Composition pharmaceutique comprenant un composé selon l'une quelconque des revendications 1 à 7, ou un sel pharmaceutiquement acceptable de celui-ci, comme principe actif, et un support ou diluant pharmaceutique.

9. Composé selon l'une quelconque des revendications 2 à 4, ou sel pharmaceutiquement acceptable de celui-ci, destiné à être utilisé comme médicament.

10. Composé selon l'une quelconque des revendications 2 à 4, ou sel pharmaceutiquement acceptable de celui-ci, destiné à être utilisé dans le traitement de la maladie d'Alzheimer.
